Europäisches Patentamt

European Patent Office

Office européen des brevets

(19)

(11) Publication number: **0 293 936 B1**

(12) **EUROPEAN PATENT SPECIFICATION**

(45) Date of publication of patent specification: **22.02.95**

(51) Int. Cl.⁶: **C12N 9/64**, C12N 15/58, A61K 38/49, C12N 1/21, C12N 5/10

(21) Application number: **88108951.0**

(22) Date of filing: **03.06.88**

(54) **Tissue plasminogen activator analogs having modified growth factor domains.**

(30) Priority: **02.03.88 US 162847**
**04.06.87 US 58061**

(43) Date of publication of application:
**07.12.88 Bulletin 88/49**

(45) Publication of the grant of the patent:
**22.02.95 Bulletin 95/08**

(84) Designated Contracting States:
**AT BE CH DE ES FR GB GR IT LI LU NL SE**

(56) References cited:
**EP-A- 0 199 574     EP-A- 0 207 589**
**EP-A- 0 213 794     WO-A-84/01786**
**WO-A-87/04722     GB-A- 2 200 640**

(73) Proprietor: **ZYMOGENETICS, INC.**
**4225 Roosevelt Way N.E.**
**Seattle, WA 98108 (US)**

Proprietor: **NOVO NORDISK A/S**
**Novo Allé**
**DK-2880 Bagsvaerd (DK)**

Proprietor: **Eisai Co., Ltd.**

**6-10, Koishikawa 4-chome**
**Bunkyo-ku**
**Tokyo 112 (JP)**

(72) Inventor: **Mulvihill, Eileen R.**
**4016 Francis Avenue**
**North Seattle**
**Washington 98103 (US)**
Inventor: **O'Hara, Patrick J.**
**4318 Winslow Place**
**North Seattle**
**Washington 98103 (US)**
Inventor: **Nexo, Bjorn A.**
**Soborg Parkhalle 10**
**DK-2860 Soberg (DK)**
Inventor: **Yoshitake, Shinji**
**221 Tsukuba Hights**
**16-2 Higashiarai**
**Yatabe-machi**
**Tsukuba-gun**
**Ibaraki (JP)**
Inventor: **Ikeda, Yasunori**
**5-28-9 Karya-cho**
**Ushiku-shi**
**Ibaraki (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Suzuki, Suguru**
**2-1 Kariya-cho**
**Ushiku-shi**
**Ibaraki (JP)**
Inventor: **Hashimoto, Akira**
**5-15-2 Matsuba**
**Ryugasaki-shi**
**Ibaraki (JP)**
Inventor: **Yuzuriha, Teruaki**
**500-6 Shimohirooka, Sakura-mura**
**Niihari-gun**
**Ibaraki (JP)**


(74) Representative: **Brown, John David et al**
**FORRESTER & BOEHMERT**
**Franz-Joseph-Strasse 38**
**D-80801 München (DE)**

## Description

Technical Field

The present invention relates to fibrinolytic agents, methods for their production, and pharmaceutical compositions containing them. More specifically, it relates to tissue plasminogen activator analogs having a modified growth factor domain.

Background Art

Hemostasis is maintained by a complex interplay of a variety of enzymes. Clotting factors interact in a "cascade" of activation steps which eventually leads to the formation of a fibrin clot. Subsequent degradation of the fibrin clot is accomplished by the fibrinolytic system, which involves the serine protease plasmin, a proteolytic enzyme which breaks down fibrin. Plasmin is a broad spectrum protease which also cleaves certain coagulation factors, thereby inactivating them. Production of plasmin from its inactive precursor, plasminogen, is mediated by tissue plasminogen activator (t-PA), a fibrin-specific serine protease which is believed to be the physiological vascular activator of plasminogen. Urokinase-type plasminogen activator (u-PA) is another member of the class of plasminogen activators characterized as serine proteases. t-PA and u-PA are functionally and immunologically distinguishable. If the normal hemostatic system becomes upset, clots may form at inappropriate times and places, leading to myocardial infarction, deep vein thrombosis, pulmonary embolism and stroke. Tissue damage resulting from these conditions may result in death or serious disability.

t-PA normally circulates as a single polypeptide chain of $M_r$ = 72,000 daltons, which is converted to a two-chain form by cleavage of a peptide bond between amino acids 275 (Arg) and 276 (Ile). The heavy chain of t-PA (two variants of $M_r$ 40,000 and 37,000) is derived from the amino-terminus, while the light chain ($M_r$ 33,000) is derived from the carboxy-terminal end of the t-PA molecule. This cleavage is catalyzed by trypsin or plasmin, and is accompanied by an increase in activity, as measured using synthetic substrates, and by an increase in fibrinolytic activity. Single-chain t-PA becomes active upon binding to fibrin, probably due to a conformational change in the activator induced by binding to fibrin. Cleavage to the two-chain form may be associated with rapid clearance of t-PA from the bloodstream, but conflicting reports on this have been published (see Wallen et al., Eur. J. Biochem. 132:681-686, 1983), and the clearance mechanism is poorly understood.

A two-dimensional model of the potential precursor t-PA protein has been established (Ny et al., Proc. Natl. Acad. Sci. USA 81:5355-5359, 1984). From this model, it was determined that the heavy chain contains two triple disulfide structures known as "kringles." Similar kringle structures also occur in prothrombin, plasminogen and urokinase, and are believed to be important for binding to fibrin (Ny et al., ibid.). The second kringle (K2) of t-PA is believed to have a higher affinity for fibrin than the first kringle (K1) (Ichinose, Takio and Fujikawa, J. Clin. Invest. 78:163-169, 1986; Verheijen et al., EMBO J. 5:3525-3530, 1986).

In addition, the heavy chain of t-PA contains a "growth factor" domain, a triple disulfide-bonded structure which has homology to epidermal growth factor and to similar domains in protein C, factor VII, factor IX and factor X. The growth factor domain of native t-PA encompasses approximately amino acids 48-90. It has been found that the growth factor domain participates in the rapid in vivo clearance of t-PA, and that deletion of the growth factor domain neither prevents the binding of the resultant molecule to fibrin nor blocks its ability to activate plasminogen.

The heavy chain of t-PA also contains a "finger" domain that is homologous to the finger domains of fibronectin. Fibronectin exhibits a variety of biological activities, including fibrin binding; its fibrin-binding activity has been correlated to four or five of its nine finger domains.

The light chain of t-PA contains the active site for serine protease activity (the serine protease domain), which is highly homologous to the active sites of other serine proteases.

The precursor form of t-PA additionally comprises a pre region followed donwstream by a pro-region, which are collectively referred to as the "pre-pro" region. The pre-region contains a signal peptide which is important for secretion of t-PA by vascular endothelial cells (Ny et al., ibid.). The pre sequence is believed responsible for secretion of t-PA into the lumen of the endoplasmic reticulum, a necessary step in extracellular secretion. The pro sequence is believed to be cleaved from the t-PA molecule following transport from the endoplasmic reticulum to the Golgi apparatus.

The use of t-PA for fibrinolysis in animal and human subjects has highlighted several shortcomings of the native molecule. The half-life of t-PA in vivo has been shown to be as short as three minutes in humans (Nilsson et al., Scand. J. Haematol. 33:49-53, 1984). Injected t-PA is rapidly cleared by the liver, and, within 30 minutes, most of the injected material is metabolized to low molecular weight forms. This short half-life may result in a need for high therapeutic dosages. Typically, native t-PA is administered at a dose of 30 to 150 mg per patient, and the low solubility of the protein necessitates prolonged infusion. Fuchs et al. (Blood 65:539-544, 1985) concluded that infused t-PA is cleared by the liver in a process independent of the proteolytic site and that infused t-PA will not accumulate in the body; that is, the clearance mechanism cannot be saturated. Furthermore, doses of t-PA sufficient to lyse coronary thrombi are far larger than normal physiological levels, and may cause activation of plasminogen throughout the body, leading to systemic degradation of fibrinogen (Sherry, ibid.), which results in dangerous bleeding episodes.

Various workers have modified t-PA in attempts to enhance its clinical suitability. Rosa and Rosa (International Patent Application WO 86/01538) modified the Lys at position 277 of t-PA to stabilize the single-chain form of t-PA. Ile (277) t-PA produced in E. coli was found to be less active as a single-chain molecule, as compared to native t-PA. Wallen et al. (ibid.) postulated that this lysine residue may be responsible for proteolytic activity of single-chain t-PA. Heyneker and Vehar (published British Patent Application 2,173,804) disclose amino acid substitutions around the cleavage site of t-PA. A variant t-PA comprising Glu at position 275 was shown to have greater specific activity, as compared to native t-PA. This variant t-PA also formed fewer complexes with t-PA inhibitor. The single-chain form was also shown to have greater affinity for fibrin than the two-chain form. Robinson (WO 84/01786) used enzymatic means to remove carbohydrate side chains from t-PA to increase plasma half-life. Lau et al. (Bio/Technology 5:953, 1987) disclose a t-PA variant lacking carbohydrate at position 451. Van Zonneveld et al. (Proc. Natl. Acad. Sci. USA 83: 4670-4674, 1986) disclose modified forms of t-PA wherein portions of the heavy chain have been deleted. Robinson et al. (EP 207,589 A1) disclose mutant forms of t-PA in which the growth factor domain has been deleted or otherwise modified. Larsen et al. (WO 87/04722) disclose t-PA-like proteins with amino acid substitutions and/or deletions. Ehrlich et al. (Fibrinolysis 1:75, 1987) disclose a recombinant t-PA molecule lacking the kringle domains. However, these variant forms of t-PA do not fully overcome the problems associated with the native protein.

There remains a need in the art for a plasminogen-activating protein with a long half-life and high specificity for fibrin. The present invention fulfills this need by providing novel derivatives of tissue plasminogen activator in which the growth factor domain has been structurally altered. The t-PA analogs described herein provide significant advantages over native t-PA as therapeutic fibrinolytic agents by permitting the use of much smaller doses, thus overcoming the problems of low solubility of native t-PA and potentially permitting administration by injection rather than infusion. Through the use of recombinant DNA technology, a consistent and homogeneous source of these proteins is provided. The proteins can be utilized to lyse existing clots in heart attack and stroke victims and in others where the need is lyse or suppress the formation of fibrin matrices is therapeutically desirable.

Disclosure of the Invention

According to the first aspect of the present invention there is provided a t-PA analog containing the growth factor domain of native t-PA, said domain having cysteine residue no. 84 of native t-PA replaced with serine. According to the second aspect of the present invention there is provided a t-PA analog containing the growth factor domain of native t-PA, said domain having cysteine residue replaced with serine, wherein the analog further contains a substitution of at least one amino acid within thirteen amino acid residues of the cleavage site, said substitution resulting in an increased resistance to cleavage by plasmin. According to a third aspect of the present invention there is provided a t-PA analog containing the growth factor domain of native t-PA, said domain having cysteine residue replaced with serine, wherein the analog contains two kringle structures, at least one of which lacks carbohydrate. According to a fourth aspect of the present invention there is provided a t-PA analog containing the growth factor domain of native t-PA, said domain having cysteine residue replaced with serine, wherein the analog contains a kringle structure derived from plasminogen. Preferably, the plasminogen kringle structure is selected from the K1, K4 and K5 kringle domains of plasminogen. Further, the analog may further contain the K2 kringle structure of native t-PA positioned downstream of the plasminogen kringle structure.

There is also provided a t-PA analog having extended half-life, which comprises the t-PA analog of native t-PA in which the cysteine residue at position no. 84 in native t-PA is replaced with serine.

DNA sequences encoding the t-PA analogs described above, as well as expression vectors containing such DNA sequences, are also disclosed. Preferred expression vectors in this regard are Zem99-9100 or Zem99-9200.

Host cells transfected or transformed with such an expression vector are also disclosed. The host cell may be E. coli or a mammalian host cell, such as BHK host cells.

Still another aspect of the present invention discloses pharmaceutical compositions comprising a t-PA analog as described herein, and a physiologically acceptable carrier or diluent.

These and other aspects of the present invention will become evident upon reference to the following detailed description and attached drawings.

Brief Description of the Drawings

Figure 1 illustrates the pre-pro t-PA coding sequence constructed from cDNA and synthesized oligonucleotides, together with the amino acid sequence of the encoded protein. Numbers above the lines refer to nucleotide position and numbers below the lines refer to amino acid position.

Figure 2 illustrates the construction of the vector Zem99.

Figure 3 illustrates the construction of the vectors Zem219b and Zem219c.

Figure 4 illustrates the construction of the vectors Zem99-9100 and Zem99-9200.

Figure 5 illustrates the nucleotide sequence of the mutant DNA sequence in Zem99-9100, together with the amino acid sequence of the encoded t-PA analog. Numbers refer to amino acid position.

Figure 6 illustrates the nucleotide sequence of the mutant DNA sequence in Zem99-9200, together with the amino acid sequence of the encoded t-PA analog. Numbers refer to amino acid position.

Figure 7 illustrates the construction of the vectors ZpL5 and ZpL7.

Figure 8 illustrates certain growth factor domain sequence substitutions, together with the combinations of oligonucleotides encoding the chimeric domains.

Figure 9 illustrates the construction of the pMH series of expression vectors comprising DNA sequences encoding plasminogen activators with altered activation sites.

Figure 10(A)-(I) illustrates the amino acid sequences of the finger domain of native t-PA and of consensus finger domains.

Figure 11 illustrates the amino acid sequence and DNA sequence of the K1 domain of plasminogen.

Figure 12 shows partial restriction maps of clones #1-3 and #8-5, which encode portions of the plasminogen K1 domain.

Figure 13 illustrates the construction of plasmid pPKA.

Figure 14 illustrates the construction of a vector containing the plasminogen K1 coding sequence.

Figure 15 illustrates the construction of plasmid Zem99-2020.

Figure 16 illustrates the construction of the plasmids Zem99-8000 and Zem99-8100.

Figures 17 and 18 show the cDNA sequences and amino acid sequences of representative t-PA analogs.

Figure 19 shows the results of a clot lysis assay on native t-PA and representative t-PA analogs of the present invention. (        ) indicates native t-PA, (----) analog #9100, (-•-) analog #9200.

Figure 20 shows a plot of plasma level vs. time for native t-PA and a representative t-PA analog which were administered to rats. Symbols used are O, analog 9100; Δ, analog 9200; and O, native t-PA.

Figure 21 shows the results of plasma half-life studies of native t-PA and three representative mutant t-PAs.

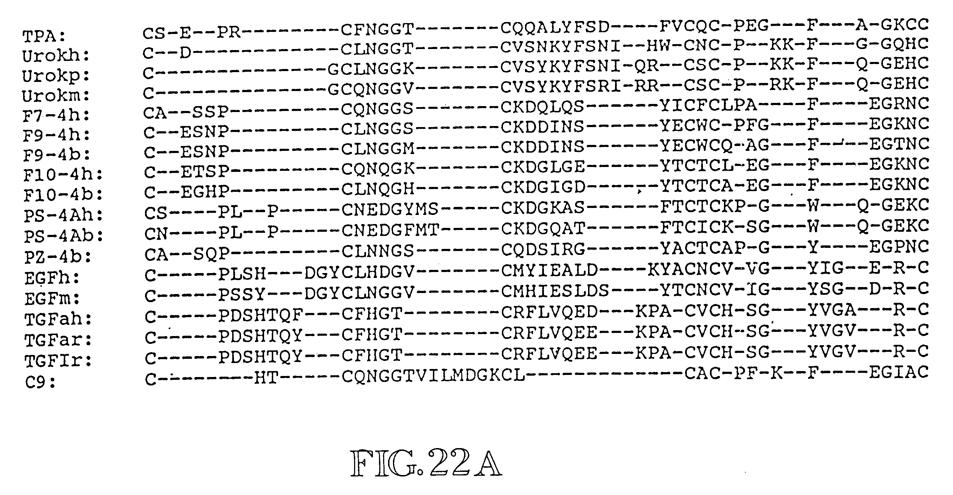

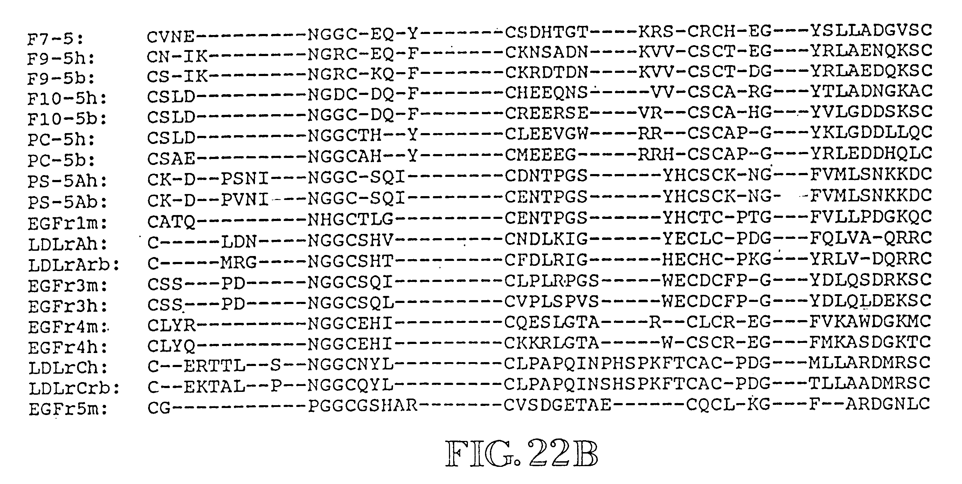

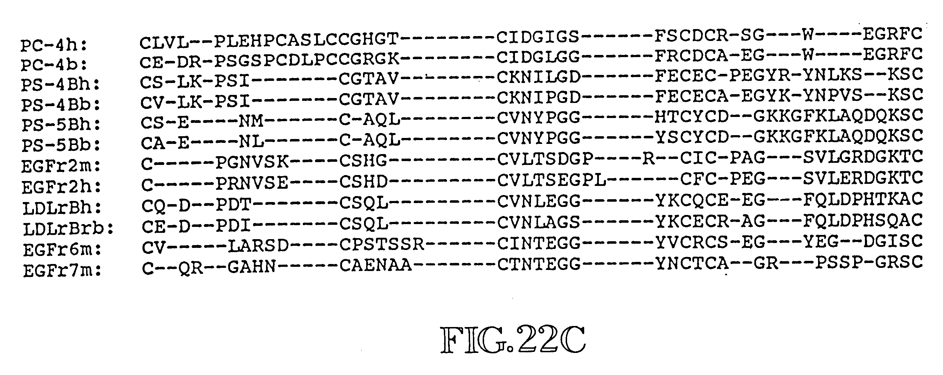

Figures 22A, B and C depict a comparison of the amino acid sequences of growth factor domains of a variety of selected proteins. The sequences are identified as follows: TPA, native tissue plasminogen activator; Urok, urokinase; F7, factor VII; F9, factor IX; F10, factor X; PS, protein S; PZ, protein Z; EGF, epidermal growth factor; TGFa, transforming growth factor alpha; TGFIr, transforming growth factor type I (rat); C9, complement factor 9; LDLr, low density lipoprotein receptor; EGFr, EGF receptor; PC, protein C. Other designations are h, human; p, porcine; m, mouse; b, bovine; and rb, rabbit. For factors VII, IX, X and proteins C, S and Z, numbers and uppercase letters refer to gene exons. For EGF receptor and LDL receptor, respectively, numbers and uppercase letters indicate a particular growth factor domain. For example, PC-4h is the human protein C fourth exon growth factor domain; LDLrBrb is growth factor domain B of rabbit low density lipoprotein receptor. Gaps (-) have been inserted to maximize the homologies among the proteins. Amino acids are designated with the standard single letter code.

Best Mode for Carrying Out the Invention

Prior to setting forth the invention, it may be helpful to an understanding thereof to set forth definitions of certain terms used herein.

Complementary DNA or cDNA: A DNA molecule or sequence which has been enzymatically synthesized from the sequences present in an mRNA template, or a clone of such a molecule.

DNA construct: A DNA molecule, or a clone of such a molecule, either single- or double-stranded, which has been modified through human intervention to contain segments of DNA combined and juxtaposed in a manner which would not otherwise exist in nature.

Plasmid or vector: A DNA construct containing genetic information which provides for its replication when inserted into a host cell. Replication may be autonomous or achieved by integration into the host genome. A plasmid generally contains at least one gene sequence to be expressed in the host cell, as well as sequences which encode functions that facilitate such gene expression, including promoters, transcription initiation sites, and transcription terminators. It may be a linear molecule or a closed, circular molecule.

Pre-pro region: An amino acid sequence which generally occurs at the amino-termini of the precursors of certain proteins, and which is generally cleaved from the protein, at least in part, during secretion. The pre-pro region comprises, in part, sequences directing the protein into the secretory pathway of the cell, and generally contains a region which is rich in hydrophobic amino acids.

Domain: A three-dimensional, self-assembling array of amino acids of a protein molecule, which contains structural elements necessary for a specific biological activity of that protein.

Biological activity: The function or set of functions performed by a molecule in a biological context (i.e., in an organism, a cell, or an in vitro facsimile thereof). Biological activities of proteins may be divided into catalytic and effector activities. Catalytic activities of fibrinolytic agents often involve the activation of other proteins through specific cleavage of precursors. In contrast, effector activities include specific binding of the biologically active molecule to other molecules, such as fibrin, or to cells. Effector activity frequently augments, or is essential to, catalytic activity under physiological conditions. Catalytic and effector activities may, in some cases, reside in the same domain of the protein. For plasminogen activators, biological activity is characterized by the conversion of the pro-enzyme or zymogen plasminogen into plasmin, which in turn degrades fibrin matrices. Because fibrin acts as a cofactor in the activation of plasminogen by t-PA, single chain t-PA has relatively little activity in the absence of fibrin.

Plasminogen Activator: A protein which, in the presence of fibrin, can convert the pro-enzyme plasminogen into plasmin.

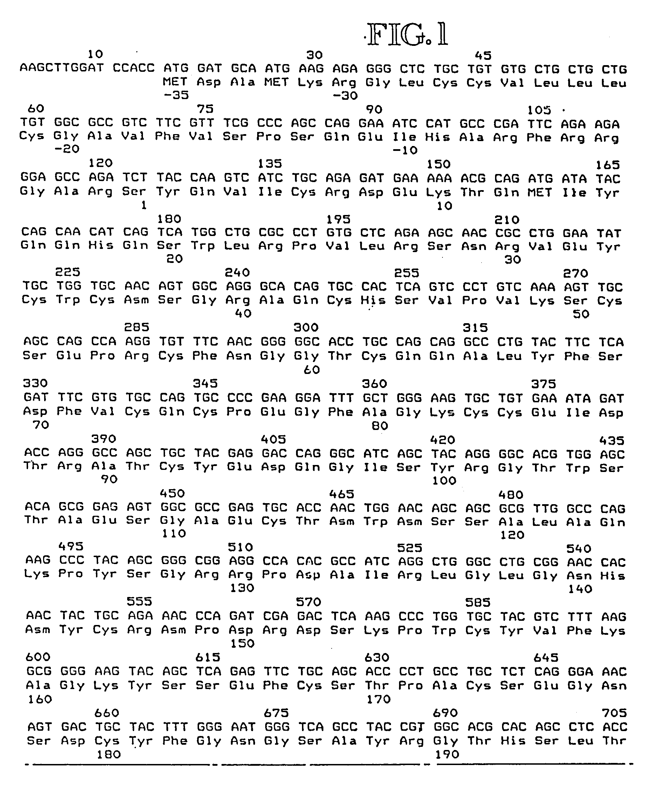

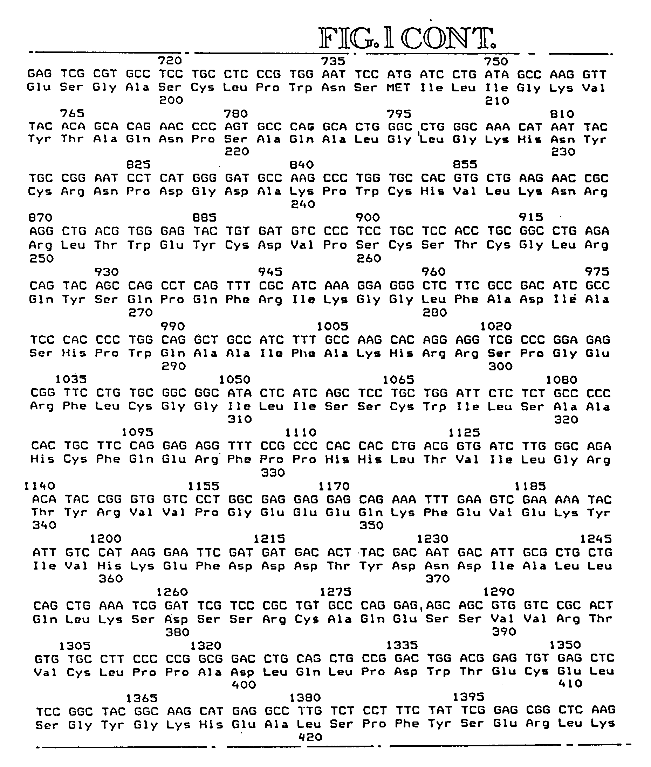

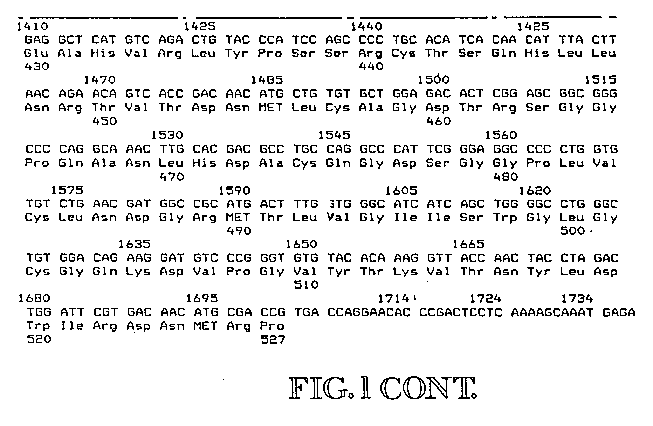

Native t-PA: A protein having the structure and biological activity of tissue plasminogen activator as isolated from human melanoma cells (see EP 0041766 A2). Native t-PA has the amino acid sequence of the melanoma cell t-PA, or may contain slight variations in sequence. Such variations, arising from, for example, genetic polymorphisms, will not substantially alter the structure or activity of the protein. The biological activity of native t-PA is characterized by the conversion of the pro-enzyme or zymogen plasminogen into plasmin, which in turn degrades fibrin matrices. Native t-PA may be isolated from cells which naturally produce it, or may be prepared from recombinant cells which have been transfected or transformed with a DNA sequence encoding native t-PA. The amino acid sequence of a representative native t-PA is shown in Figure 1.

t PA analog: A protein having the characteristic biological activity of plasminogen activators as defined above, further characterized by the presence of a specific artificially induced mutation in the amino acid sequence. The DNA sequence encoding a t-PA analog is referred to as a "mutant DNA sequence," and will generally be in the form of a cDNA. The term "specific artifically induced mutation" includes deletions, insertions and substitutions in the amino acid sequence, which may be introduced through manipulation of a cloned DNA sequence. In general, the biological activity of the t-PA analogs will be measurably altered from that of native t-PA.

Plasma half-life: The period of time required for the elimination or inactivation of 50% of a substance from the bloodstream of an animal. For t-PA, plasma half life is measured by monitoring the disappearance of t-PA from the bloodstream of a test animal, such as a rat, using, for instance, an enzyme-linked immunosorbent assay (ELISA).

As noted above, human t-PA is a 72,000 dalton protein which may exist in a two-chain form. The protein contains a number of structural domains which individually and collectively contribute to the biological activity of the protein. One of these domains is called the "growth factor domain" (hereinafter "GF domain") due to its homology with epidermal growth factor (EGF). The entire mature protein contains 35 cysteine residues, 34 of which participate in inter- or intrachain disulfide bonds (Ny et al., ibid.). Seven of these cysteines are within the GF domain and are arranged in three disulfide bonds, with the seventh cysteine remaining upaired.

Also as noted above, the GF domain has been found to be responsible for certain limitations in the use of native t-PA as a pharmaceutical agent. Investigations by the inventors have produced data indicating that the GF domain may be partially responsible for the rapid clearing of t-PA from the bloodstream.

Furthermore, the presence of a free sulfhydryl group in the GF domain may destabilize the protein. In general, free sulfhydryl groups of proteins in solution tend to be oxidized spontaneously, and may also form intermolecular disulfide bridges, leading to protein aggregation. When a protein having a free sulfhydryl group is used as a pharmaceutical agent, these changes in physiochemical properties, resulting from free sulfhydryl groups, may alter the immunogenic or antigenic properties of the protein and therefore limit its therapeutic utility. This effect has been observed for interleukin-2 (Wang et al., Science 224:1431-1433, 1984. and Liang et al., J. Biol. Chem. 261:334-337, 1986).

To have both fibrin binding activity and serine protease activity, the plasminogen activators will contain at least a GF domain, a kringle domain and a serine protease domain. Preferably, the kringle domain will be the $K_2$ domain of native t-PA. Additional kringle domains may also be included as discussed in more detail below. It is also advantageous to include a finger domain in order to provide greater affinity for fibrin. A preferred finger domain is that of native t-PA.

The inventors have discovered that by altering the structure of the GF domain of t-PA, the plasma half-life of the protein may be prolonged. One such alteration involves the climination of a cysteine residue from the GF domain of t-PA. Preferably, the cysteine residue at position 84 is removed, although the other cysteine residues at positions 51, 56, 62, 73, 75 and 83 may also be removed, either singly or in combination. In any event, it is preferable that the resulting molecule not have an unpaired cysteine, for reasons discussed above. Cysteine residues may be removed by deletion or amino acid substitution, with substitution being the preferred method. In principle, any amino acid may be substituted for cysteine, although serine and alanine are preferred. A particularly preferred substitute amino acid is serine. Other modifications of the GF domain sequence include the substitution of a plurality of substantially consecutive amino acids of the GF domain of native t-PA. Amino acid substitutions are made in this region with the goal of disrupting the region involved in the rapid clearance of t-PA from plasma without drastically altering the potential receptor region located within the growth factor domain. The loop structure bounded by amino acids 62 and 73 is a primary candidate for the receptor binding region due to its low homology with corresponding regions in other growth factor domain-containing proteins and characteristics which suggest that it is located on the protein surface. It is preferred that substitute amino acids be selected from those found in corresponding positions in growth factor domains of other proteins. Targeted changes may eliminate specific receptor interactions without destroying those features which allow the domain to assume its overall EGF-like conformation, including disulfide bonding between cysteine pairs 51 and 62, 56 and 73, and 75 and 84. In general, it is desirable to maintain the ability of this region to act as a growth factor domain. Toward this end, amino acid substitutions are chosen so as to avoid major changes in conformation but with the goal of changing the chemical nature of one or more amino acid residues at specific positions. For example, it may be desirable to retain certain highly conserved features of growth factor domains. As shown in Figure 22, these features may include (a) the presence of the sequence Asn-Gly-Gly (NGG) proximate to the second cysteine residue of the t-PA growth factor domain; and (b) the presence of the sequence Cys-X-Cys, where X is any amino acid, corresponding to cysteine residues four and five of the native t-PA growth factor domain.

Preferred substitutions include the substitution of a plurality of substantially consecutive amino acids from the growth factor domain(s) of one or more proteins with a relatively long plasma half-life, such as coagulation factors VII and IX. In addition, more than one region of the growth factor domain of native t-PA may be replaced by the respective blocks of amino acids discussed above. Further, one can substitute a complete growth factor domain from another protein. Other substitutions are made by designing consensus growth factor sequences based on sequences in factor VII, factor IX, protein C, epidermal growth factor and other proteins containing a growth factor domain. Growth factor domains are also found in urokinase, factor X, protein S, protein Z, low density lipoprotein receptor, transforming growth factor, epidermal growth factor receptor, thrombomodulin and thrombospondin. A particularly preferred such consensus sequence is the amino acid sequence methionine-glutamic acid-glycine-asparagine-histidine-leucine-alanine-asparagine (MEGNHLAN), which was substituted for amino acids 63-70 of native t-PA. In certain preferred embodiments,the cysteine residue corresponding to amino acid residue 83 of the native t-PA GF domain is also replaced with another amino acid in order to increase the stability of the resultant molecule. In principle, any amino acid may be substituted for cysteine, although serine and alanine are preferred. A particularly preferred substitute amino acid is serine.

In addition to the alterations in the GF domain, the t-PA analogs of the present invention may contain additional mutations. These mutations may be amino acid substitutions, deletions or additions. Many such mutations have been described, as discussed above. Particularly preferred mutations include substitutions of the finger and/or K1 domains, amino acid substitutions near the cleavage (activation) site, the removal of the carbohydrate attachment site in the K1 domain or the modification of the carbohydrate attachment site in the K2 domain. Such mutations will enhance the clinical suitability of the t-PA analogs. For example, the K1 domain of native t-PA may be replaced with a kringle domain derived from another protein to enhance the fibrin specificity of the resulting analog. Suitable kringle domains in this regard include the K1, K2, K3, K4 and K5 domains of plasminogen, the K2 domain of native t-PA, the K1 and K2 domains of prothrombin, and the kringle domain of factor XII. The K1 domain of plasminogen is particularly preferred. Modifications to the activation site may increase clot specificity of the t-PA analog by reducing its sensitivity to proteolytic cleavage by plasmin and/or allowing cleavage by thrombin. Modifications of carbohydrate attachment sites increase the uniformity of the protein product and increase plasma half-life. By altering the Asn residues at the N-linked glycosylation sites (sequence Asn-X-Ser/Thr, wherein X is any amino acid) to another amino acid residue, glycosylation may be blocked. It is particularly preferred that the Asn residues be changed to Gln residues. Asn may also be changed to Ser or Thr. Additionally, other alterations in the sequence may be introduced for the purpose of blocking glycosylation of the encoded protein. For example, a proline residue in the second position of the sequence Asn-X-Ser/Thr may prohibit glycosylation (Marshall, Biochem. Soc. Symp. 40:17-26, 1974). Other substitutions may also be made at this position. The third amino acid of an N-linked glycosylation site may also be changed, preferably to Ala, Cys, Gly, Asn or Gln. Glycosylation sites may be changed individually or in combination. In one preferred embodiment, carbohydrate addition at Asn-117 is blocked by the substitution of a glutamine residue.

According to the present invention, it is preferred to produce these novel proteins through the use of recombinant DNA technology, using cDNA clones or genomic clones as starting materials. Suitable DNA sequences can also be synthesized according to standard procedures, including automated synthesis, or may be obtained from commercial sources. It is preferred to use cDNA clones because, by employing the full-length cDNA encoding native t-PA as starting material for producing modified t-PA, introns are removed so that all exons of the native t-PA are present and correctly oriented with respect to one another. The cDNA can also be used as a template for deletion, alteration or insertion of sequences via oligonucleotide-directed mutagenesis.

Recombinant DNA technology allows the convenient enhancement of the fibrin-binding domain of native t-PA. Such enhancements may be achieved by the insertion of additional kringle structures, modification of kringle structures, the addition of finger domains, or the substitution of the finger domain. This methodology provides, a means for selecting the optimum combination of functional domains found in native t-PA or in related proteins, and thus provides fibrinolytic agents with enhanced biological activity with respect to fibrin binding and specificity of serine protease activity.

Amino acid substitutions or deletions are introduced by site-specific mutagenesis using the cloned t-PA sequence or a portion thereof as a template. Techniques for oligonucleotide-directed in vitro mutagenesis are generally known in the art. A preferred such method is that of Zoller and Smith (DNA 3:479-488, 1984).

Amino acid substitutions are preferably made by synthesizing oligonucleotides of convenient length, preferably about 30 to 50 nucleotides in length, and assembling them to construct the desired coding sequence. Oligonucleotides of this length permit the design of modular sequences which can then be arranged in a variety of combinations in order to obtain a large number of overall sequences. Such combination is facilitated by designing the oligonucleotides so that when paired, the resulting fragment ends are blunt or complementary to those of oligonucleotide pairs encoding adjacent sequences. It will be understood, however, that complete GF substitutions can be constructed using full-length oligonucleotides. Oligonucleotides provide the added advantage of permitting codon use optimization appropriate to the chosen host cell type.

Alternatively, DNA sequences encoding the GF substitutions may be obtained from cDNA or genomic clones encoding proteins having GF domains. Clones of factor IX (Kurachi and Davie, Proc. Natl. Acad. Sci. USA 79:6461-6464, 1982; Anson et al, EMBO J. 3:1053-1060, 1984), factor VII (Hagen et al., Proc. Natl. Acad. Sci. USA 83:2412-2416, 1986), protein C (Foster et al., Proc. Natl. Acad. Sci. USA 82:4673-4677, 1985), and epidermal growth factor (Gray et al., Nature 303:722-725, 1983) have been described. Suitable clones encoding these and other proteins may be obtained by procedures familiar to those having ordinary skill in the genetic engineering art.

The sequences encoding the GF domain substitutions are then inserted into the t-PA sequence at convenient restriction sites. In some cases it will be necessary to use in vitro mutagenesis to properly join the sequences.

The mutated sequence is, if necessary, joined to the remainder of the t-PA coding sequence, and the complete coding sequence is then inserted into an expression vector. The mutant sequences may be expressed in various host cells, including mammalian cells, yeast and other fungi, and bacteria.

Production of recombinant t-PA in bacteria, yeast and mammalian cells is disclosed by, for example, Goeddel et al. (EP 93619 A1), Meyhack and Hinnen (EP 143,081 A2), and Gill (EP 174,835 A1). Methods for transfecting mammalian cells and for transforming bacteria and fungi with foreign DNA are well known in the art. Suitable expression vectors will comprise a promoter which is capable of directing the transcription of a cloned DNA sequence in a host cell and a functional transcription termination site.

In some instances, it is preferred that expression vectors further comprise an origin of replication, as well as sequences which regulate and/or enhance expression levels, depending on the host cell selected. Suitable expression vectors may be derived from plasmids, RNA and DNA viruses or cellular DNA sequences, or may contain elements of each.

Preferred prokaryotic hosts for use in carrying out the present invention are strains of the bacteria Escherichia coli, although Bacillus and other genera are also useful. Techniques for transforming these hosts, and for expressing foreign DNA sequences cloned in them, are well known in the art (see, for example, Maniatis et al., Molecular Cloning: A Laboratory Manual, Cold Spring Harbor Laboratory, 1982). Vectors used for expressing foreign DNA in bacterial hosts will generally contain a selectable marker, such as a gene for antibiotic resistance, and a promoter which functions in the host cell. Appropriate promoters include the trp (Nichols and Yanofsky, Meth. in Enzymology 101:155, 1983), lac (Casadaban et al., J. Bact. 143:971-980, 1980), TAC (Russell et al., Gene 20:231-243, 1982), and phage λ promoter systems. Plasmids useful for transforming bacteria include pBR322 (Bolivar et al., Gene 2:95-113, 1977), the pUC plasmids (Messing, Meth. in Enzymology 101:20-78, 1983; and Vieira and Messing, Gene 19:259-268, 1982), pCQV2 (Queen, J. Mol. Appl. Genet. 2:1-10, 1983), and derivatives thereof.

Eukaryotic microorganisms, such as the yeast Saccharomyces cerevisiae, or filamentous fungi including Aspergillus, may also be used as host cells. Particularly preferred species of Aspergillus include A. nidulans, A. niger, A. oryzae, and A. terreus. Techniques for transforming yeast are described by, for example, Beggs (Nature 275:104-108, 1978). Aspergillus species may be transformed according to known procedures, for example, that of Yelton et al. (Proc. Natl. Acad. Sci. USA 81:1740-1747, 1984). Expression vectors for use in yeast include YRp7 (Struhl et al., Proc. Natl. Acad. Sci. USA 76:1035-1039, 1979), YEp13 (Broach et al., Gene 8:121-133, 1979), pJDB248 and pJDB219 (Beggs, ibid.), and derivatives thereof. Such vectors will generally comprise a selectable marker, such as the nutritional marker TRP1, which allows selection in a host strain carrying a trp1 mutation. Preferred promoters for use in yeast expression vectors include promoters from yeast glycolytic genes (Hitzeman et al., J. Biol. Chem. 255:12073-12080, 1980; Alber and Kawasaki, J. Mol. Appl. Genet. 1:419-434, 1982; Kawasaki, U.S. Patent 4,599,311) or alcohol dehydrogenase genes (Young et al., in Genetic Engineering of Microorganisms for Chemicals, Hollaender et al., eds., p. 335, Plenum, New York, 1982; and Ammerer, Meth. in Enzymology 101:192-201, 1983). To facilitate purification of a modified t-PA protein produced in a yeast transformant and to obtain proper disulphide bond formation, a signal sequence from a yeast gene encoding a secreted protein may be substituted for the t-PA pre-pro sequence. A particularly preferred signal is the pre-pro region of the MFα1 gene (Kurjan and Herskowitz, Cell 30:933-943, 1982; and Singh, EP 123,544).

Higher eukaryotic cells may also serve as host cells in carrying out the present invention. Cultured mammalian cells, such as the BHK, CHO, NS-1, SP2/0 and J558L cell lines, are preferred. These and other cell lines are widely available, for example, from the American Type Culture Collection. A particularly preferred adherent cell line is the BHK cell line tk⁻ts13 (Waechter and Baserga, Proc. Natl. Acad. Sci. USA 79:1106-1110, 1982), hereinafter referred to as "tk⁻BHK cells." Expression vectors for use in mammalian cells will comprise a promoter capable of directing the transcription of a cloned gene introduced into a mammalian cell. Particularly preferred promoters include the SV40 promoter (Subramani et al., Mol. Cell Biol. 1:854-64, 1981) the MT-1 promoter (Palmiter et al., Science 222:809-814, 1983), and the mouse kappa gene promoter (Bergmann et al., Proc. Natl. Acad. Sci. USA 81:7041-7045, 1984). Also contained in the expression vectors is a transcription terminator, located downstream of the insertion site for the DNA sequence to be expressed. A preferred terminator is the human growth hormone (hGH) gene terminator (DeNoto et al., Nuc. Acids Res. 9:3719-3730, 1981). In addition, vectors will preferably contain enhancer sequences appropriate to the particular host cell line.

For expression of mutant t-PAs in cultured mammalian cells, expression vectors containing cloned t-PA sequences are introduced into the cells by appropriate transfection techniques, such as calcium phosphate-mediated transfection (Graham and Van der Eb, Virology 52:456-467, 1973; as modified by Wigler et al., Proc. Natl. Acad. Sci. USA 77:3567-3570, 1980; or as described by Loyter et al., Proc. Natl. Acad. Sci. USA 79:422, 1982) or electroporation (Neumann et al., EMBO J. 1:841-845, 1982). A portion of the cells take up the DNA and maintain it inside the cell for several days. A small fraction of the cells integrate the DNA into the genome of the host cell or maintain the DNA in non-chromosomal nuclear structures. These transfectants can be identified by cotransfection with a gene that confers a selectable phenotype (a selectable marker). Preferred selectable markers include the DHFR gene, which imparts cellular resistance to methotrexate (MTX), an inhibitor of nucleotide synthesis; or the bacterial neomycin resistance gene, which confers resistance to the drug G-418, an inhibitor of protein synthesis. After the host cells have taken up the DNA, drug selection is applied to select for a population of cells that are expressing the selectable marker at levels high enough to confer resistance. Selectable markers may be carried on the same vector as the sequence encoding the t-PA analog, or may be carried on a separate vector.

When using methotrexate selection, coamplification can be used to increase expression levels by adding high concentrations of MTX to the culture medium at the time of the initial selection, or by sequentially increasing the concentration of MTX in the medium, followed by repeated cloning by dilution of the drug-resistant cell lines. Variations exist in the ability to amplify and relate both to the initial genomic configuration (i.e., extra-chromosomal vs. chromosomal) of the cotransfected DNA sequences and to the mechanism of amplification itself, in which variable amounts of DNA rearrangements can occur. This is noticed upon further amplification of clones which have been previously shown to be stable. For this reason, it is necessary to clone by dilution after every amplification step. Cells which express the DHFR marker are then selected and screened for production of t-PA. Screening may be done by enzyme-linked immunosorbent assay (ELISA) or by biological activity assays.

The plasminogen activators of the present invention exhibit a fibrinolytic effect which is equivalent to that of native t-PA. However, these proteins exhibit an advantage over native t-PA in that they may have a plasma half-life as much as five times as long as that of native t-PA, suggesting that they may be superior therapeutic agents.

The plasminogen activators of the present invention may be used within pharmaceutical compositions for the treatment of thrombosis. The pharmaceutical compositions will comprise the a plasminogen activator in combination with a carrier or diluent, such as sterile water or sterile saline, and may also comprise appropriate excipients and/or solvents. The resulting aqueous solutions may be packaged for use or filtered under aseptic conditions and lyophilized, the lyophilized preparation being combined with a sterile aqueous solution prior to administration.

Typically, an aqueous solution containing 3 g of mannitol and $10^6$ units of the t-PA analog is prepared under sterile conditions. One ml aliquots of this solution are pipetted into small vials, which are then lyophilized and scaled. For injection, the lyophilized material is combined with 2 ml of sterile water, the water being provided in a sealed ampoule. Administration is preferably by injection. The proteins of the present invention will typically be administered at doses of from about 6 mg to about 100 mg per patient, depending on the weight of the patient and the nature of the thrombus to be dissolved. However, the present invention is not restricted to the above range and the dose may be varied depending on the condition. Determination of proper dose will be apparent to the skilled practitioner.

The following examples are offered by way of illustration, and not by way of limitation.

EXAMPLES

Example 1 - Construction of a Full-Length t-PA Clone

The sequence of a native human t-PA cDNA clone has been reported (Pennica et al., Nature 301:214-221, 1983). The sequence encodes a pre-pro peptide of 32-35 amino acids followed by a 527-530 amino acid mature protein.

A cDNA clone comprising the coding sequence for mature t-PA was constructed using as starting material mRNA from the Bowes melanoma cell line (Rijken and Collen, J. Biol. Chem. 256:7035-7041, 1981). This cDNA was then used to construct the plasmid pDR1296. Escherichia coli strain JM83 transformed with pDR1296 has been deposited with the American Type Culture Collection under Accession No. 53347.

Because the pre-pro sequence was not present in the cDNA clone pDR1296, it was constructed from synthesized oligonucleotides and subsequently joined to the cDNA. In the synthesized t-PA pre-pro sequence, cleavage sites for Bam HI and Nco I were introduced immediately 5′ to the first codon (ATG) of the pre-pro sequence, and a Bgl II (Sau 3A, Xho II) site was maintained at the 3′ end of the pre-pro sequence. The naturally-occurring pre-pro sequence lacks a convenient restriction site near the middle; however, the sequence GGAGCA (coding for amino acids -20 and -19, Gly-Ala) can be altered to GGCGCC to provide a Nar I site without changing the amino acid sequence.

To construct the pre-pro sequence, the following oligonucleotides were synthesized using an Applied Biosystems Model 380-A DNA synthesizer:

ZC131:    $^{5′}$GGA TCC ATG GAT GCA ATG AAG AGA GGG CTC TGC TGT CTG$^{3′}$
ZC132:    $^{5′}$TGG CGC CAC ACA GCA GCA GCA CAC AGC AGAG$^{3′}$
ZC133:    $^{5′}$GGC GCC GTC TTC GTT TCG CCC AGC CAG GAA ATC CATG$^{3′}$

ZC134:     $^{5'}$AGA TCT GGC TCC TCT TCT GAA TCG GGC ATG GAT TTC CT$^{3'}$

Following purification, oligomers ZC131 and ZC132 were annealed to produce an overlap of 12 base pairs (Section 1). Oligomers ZC133 and ZC134 were similarly annealed (Section 2). The oligomers were mixed in Pol I buffer (Bethesda Research Labs), heated to 65°C for five minutes, and slowly cooled to room temperature for four hours to anneal. Ten units of DNA polymerase I were added and the reaction proceeded for two hours at room temperature. The mixtures were electrophoresed on an 8% polyacrylamide-urea sequencing gel at 1,000 volts for 2½ hours in order to size fractionate the reaction products. The correct size fragments (those in which the polymerase reaction went to completion) were cut from the gel and extracted.

After annealing, Section 1 was cut with Bam HI and Nar I and cloned into Bam HI + Nar 1 - cut pUC8 (Vieira and Messing, Gene 19:259-268, 1982; and Messing, Meth. in Enzymology 101:20-77, 1983). Section 2 was reannealed and cut with Nar I and Bgl II and cloned into Bam HI + Nar I - cut pUC8. Colonies were screened with the appropriate labeled oligonucleotides. Plasmids identified as positive by colony hybridization were sequenced to verify that the correct sequence had been cloned.

Section 1 was then purified from a Bam HI + Nar I double digest of the appropriate pUC clone. Section 2 was purified from a Nar I + Xho II digest. The two fragments were joined at the Nar I site and cloned into Bam HI - cut pUC8.

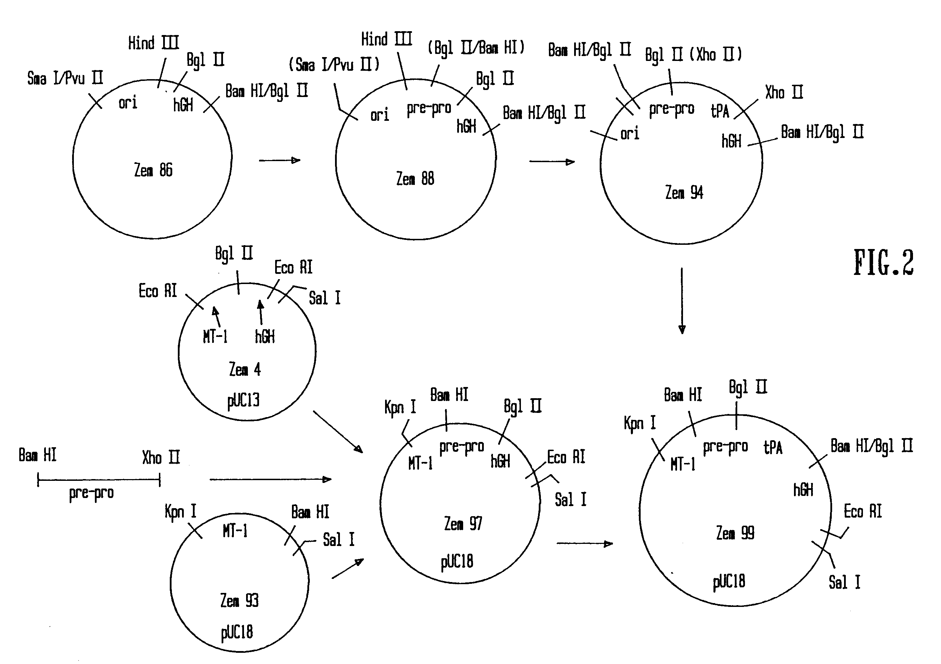

The t-PA sequence of pDR1296 was then joined to the synthesized pre-pro sequence in the following manner (Figure 2). Plasmid pIC19R (Marsh et al., Gene 32:481-486, 1984) was digested with Sma I and Hind III. The ori region of SV40 from map position 270 (Pvu II) to position 5171 (Hind III) was then ligated to the linearized pIC19R to produce plasmid Zem67. This plasmid was then cleaved with Bgl II, and the terminator region from the human growth hormone gene (De Noto et al., Nuc. Acids Res. 9:3719-3730, 1981) was inserted as a Bgl II-Bam HI fragment to produce plasmid Zem86. The synthesized t-PA pre-pro sequence was removed from the pUC8 vector by digestion with Bam HI and Xho II. This fragment was inserted into Bgl II-digested Zem86 to produce plasmid Zem88. Plasmid pDR1296 was digested with Bgl II and Bam HI and the t-PA cDNA fragment was isolated and inserted into Bgl II - cut Zem88. The resultant plasmid was designated Zem94.

The vector Zem99, comprising the MT-1 promoter, complete t-PA coding sequence, and the hGH terminator, was then assembled in the following manner (Figure 2). A Kpn I-Bam HI fragment comprising the MT-1 promoter was isolated from MThGH111 (Palmiter et al., Science 222:809-814, 1983) and inserted into pUC18 to construct Zem93. Plasmid MThGH112 (Palmiter et al., ibid.) was digested with Bgl II and re-ligated to eliminate the hGH coding sequence. The MT-1 promoter and hGH terminator were then isolated as an Eco RI fragment and inserted into pUC13 to construct Zem4. Zem93 was then linearized by digestion with Bam HI and Sal I. Zem4 was digested with Bgl II and Sal I, and the hGH terminator was purified. The t-PA pre-pro sequence was removed from the pUC8 vector as a Bam HI-Xho II fragment. The three DNA fragments were then joined, and a plasmid having the structure of Zem97 (Figure 2) was selected. Zem97 was cut with Bgl II and the Xho II t-PA fragment from Zem94 was inserted. The resultant vector is Zem99.

Example 2 - Construction of Zem219c

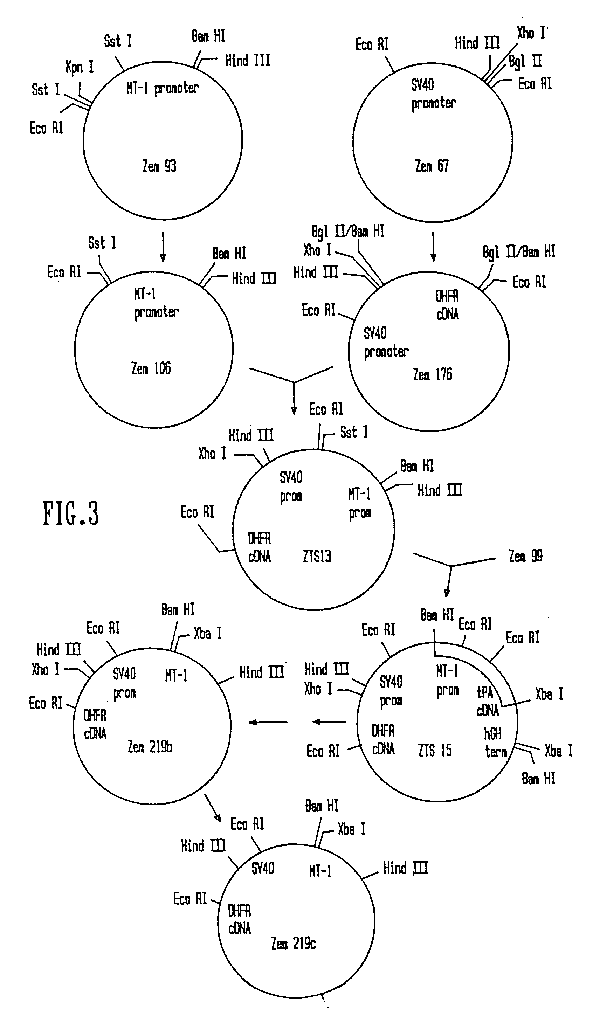

The vector Zem219c was constructed as shown in Figure 3. Plasmid pSV2-DHFR (Subramani et al., ibid.) was digested with Cfo 1, and the fragment containing the DHFR cDNA and the 3' attached SV40 sequences was isolated, repaired, and ligated to Bam HI linkers. After digestion with Bam HI, an approximately 800 bp fragment containing the entire cDNA and the SV40 terminator region was, purified and ligated to Bam HI-digested pUC8. Zem67 (Example 1) was digested with Bgl II and ligated with the Bam HI DHFR-SV40 fragment to generate plasmid Zem176. Plasmid Zem93 was digested with Sst I and re-ligated to generate plasmid Zem106, in which approximately 600 bp of sequence 5' to the MT-1 promoter were eliminated. Plasmid Zem106 was digested with Eco RI and ligated to the Eco RI fragment containing the DHFR gene from plasmid Zem176. The resulting plasmid was designated Zts13. Plasmid Zts13 was digested with Bam HI and ligated to the Bam HI fragment from plasmid Zem99 containing the entire t-PA coding region and hGH terminator sequence. The resulting plasmid was designated Zts15. Zts15 was partially digested with Bam HI, repaired and re-ligated to generate plasmid Zem219, in which the 3' Bam HI site was destroyed. Plasmid Zem219 was partially digested with Xba I, repaired and re-ligated to generate plasmid Zem219a, in which the 3' Xba I site was destroyed. Plasmid Zem219a was digested with Bam HI and Xba I, the vector sequences purified away from the t-PA cDNA sequences, and ligated with an oligomeric Bam HI-Xba I adaptor to generate the vector Zem219b. The Xho I site within the polylinker region of the SV40-DHFR sequence of Zem219b was destroyed by digestion with Xho I, repair and religation of the newly blunted ends. The resulting plasmid was designated Zem219c.

Example 3 - Replacement of Cys (83)

The t-PA coding sequence in Zem99 was mutagenized to encode a serine at position 83 (amino acid numbers refer to the sequence shown in Figure 1). Zem99 was digested with Bam HI, and a 2.4 kb fragment comprising the t-PA coding sequence and the hGH terminator was isolated. This fragment was joined to Bam HI-digested M13mp18 (obtained from Pharmacia), and the resultant recombinant phage was used to transfect E. coli JM103. A phage clone having the desired insertion was designated M13mp18/Bam-Zem99.

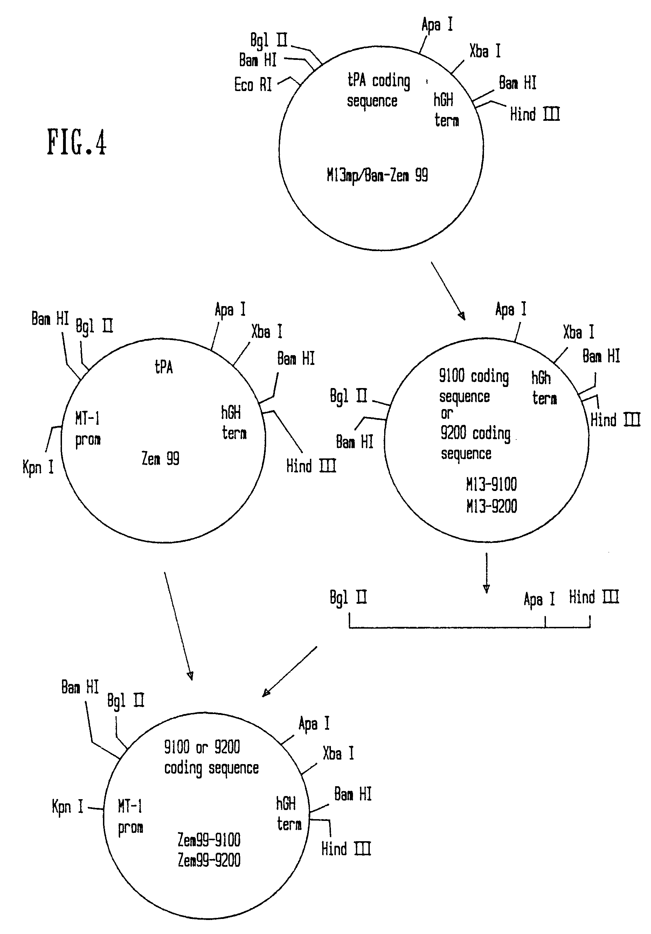

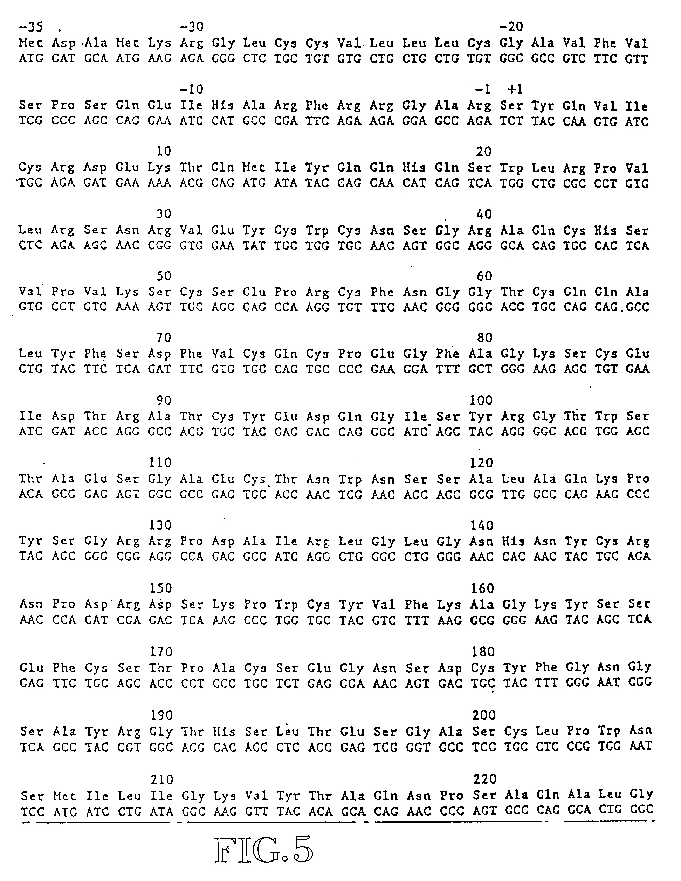

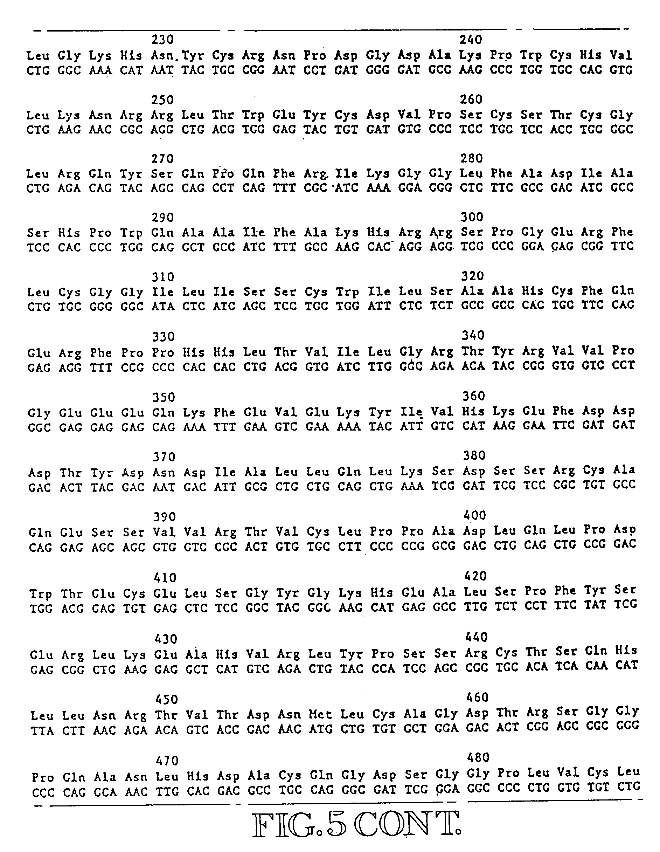

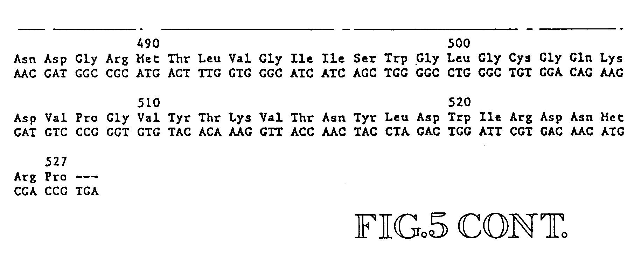

For site-specific mutagenesis, an oligonucleotide (sequence 5′ CT GGT ATC GAT TTC ACA GCT CTT CCC AGC A 3′) was synthesized and used as a mutagenic primer. The oligonucleotide was annealed to single-stranded M13mp18/Bam-Zem99. Mutagenesis was carried out according to standard procedures and single-stranded DNA was isolated for sequencing. The replicative form of the mutagenized phage, designated M13-9100RF, was digested with Bgl II and Hind III. A 2.3 kb fragment containing the t-PA sequence was recovered and joined to Zem99, which had been digested with Bgl II and Hind III (Figure 4), and the DNA was used to transform E. coli TB1. A plasmid having the desired sequence alteration was recovered and designated Zem99-9100. The mutated t-PA sequence of Zem99-9100 and the encoded amino acid sequence are shown in Figure 5.

Plasmids Zem99-9100 and pSV2-dhfr (Subramani et al., ibid.) were used to transfect tk⁻BHK cells by the method of Loyter (Proc. Natl. Acad. Sci. USA 79:422, 1982). Transformants were subjected to cloning by the limiting dilution method. The mutant protein, designated 9100, was purified from the cell culture media by affinity purification.

An E. coli TB1 transformant containing plasmid Zem99-9100 has been deposited with the Fermentation Research Institute, Agency of Industrial Science and Technology, Ministry of International Trade and Industry, Japan (FR1), under Accession No. FERM P-9269.

Example 4 - Replacement of Cys (84)

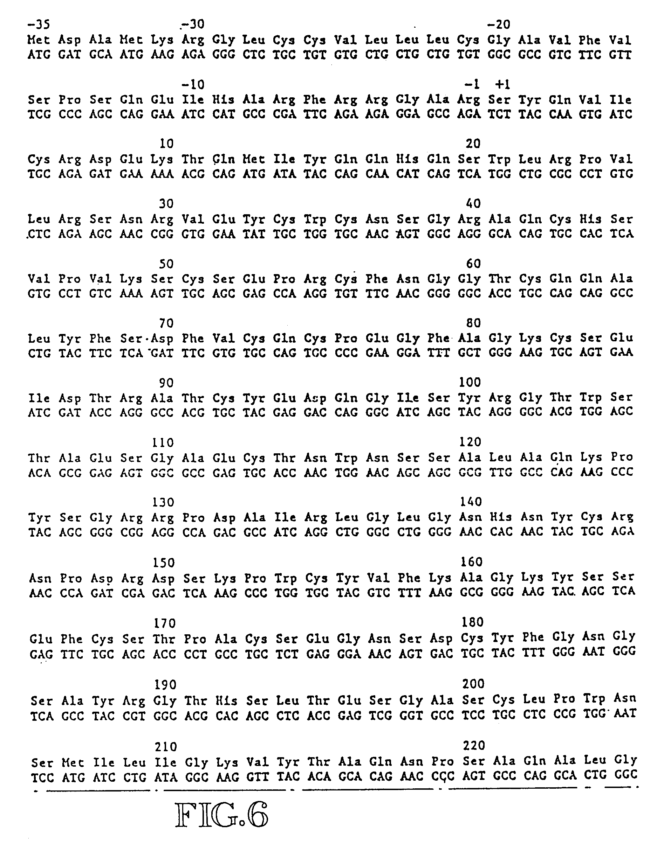

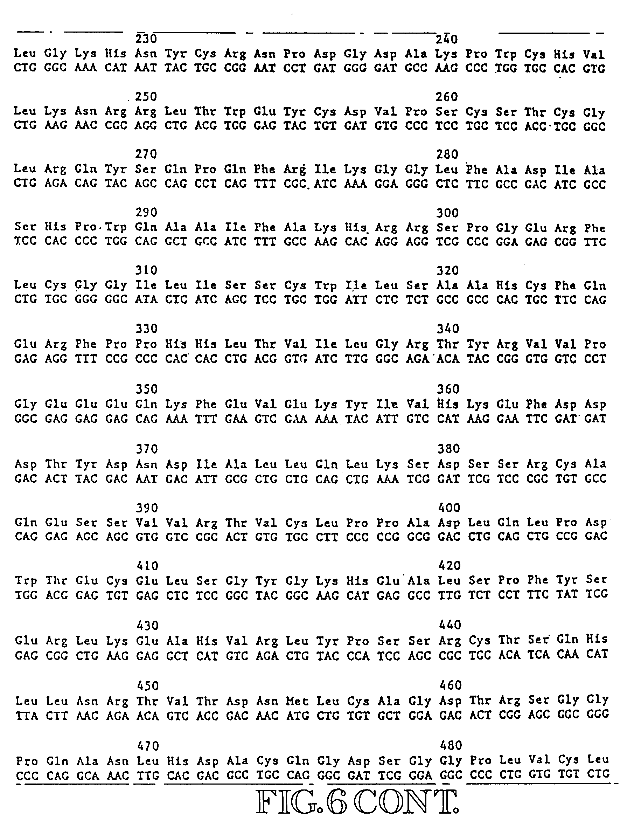

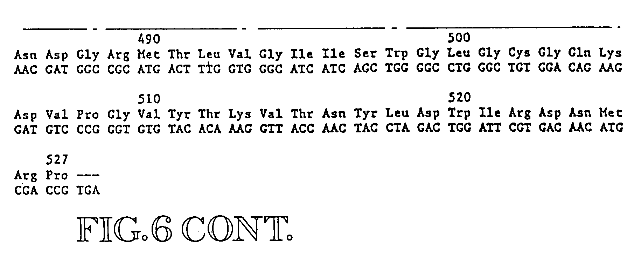

The t-PA DNA sequence was mutagenized to encode serine at amino acid 84 by means of site-specific mutagenesis using the oligonucleotide 5′ CCT GGT ATC GAT TTC ACT GCA CTT CCC 3′. The oligonucleotide was annealed to M13mp18/Bam-Zem99 and mutagenesis was carried out using standard procedures. Single-stranded mutagenized phage were sequenced and a clone having the desired sequence alternation was selected. Replicative form DNA was prepared (designated M13-9200RF) and digested with Bgl II and Hind III. The 2.3 kb t-PA fragment was isolated and joined to the Bgl II + Hind III - cut Zem99. The resultant vector was designated Zem99-9200 (Figure 4). The altered t-PA coding sequence of Zem99-9200 and the encoded amino acid sequence are shown in Figure 6.

Zem99-9200 and pSV2-dhfr were used to co-transfect tk⁻BHK cells by the method of Loyter (ibid). Transformants were subjected to cloning by the limiting dilution method. The mutant protein (9200) was purified by affinity purification.

An E. coli RR1 transformant containing plasmid Zem99-9200 has been deposited with FRI under Accession No. FERM P-9274.

Example 5 - Mutagenesis of the t-PA Growth Factor Domain

In order to replace the authentic t-PA GF domain with alternative growth factor sequences, the native t-PA sequence was first modified to place a Kpn I site between the finger and growth factor domain coding sequences. Plasmid Zem99 was digested with Bam HI and Eco RI and the 5′ t-PA fragment was recovered and inserted into Bam HI + Eco RI cut M13mp18. Phage DNA was prepared and 100 µl of the DNA solution was used to infect E. coli RZ1032 in 100µl of YT medium supplemented with 0.1 µg/ml uridine. This culture was incubated at 37°C, with vigorous shaking, overnight. Growing the M13 in RZ1032 produces phage containing uridine which are viable in RZ1032 but not in JM101.

The cells were spun out, and the phage supernatant was used to reinfect E. coli RZ1032. This second passage was performed to dilute out any JM101-derived phage which contained no uracil. Again, the cells were spun out and the phage were plated on JM101 and RZ1032. Normal viability was observed on RZ1032 plates (indicating phage at $10^9$ pfu/ml), but no plaques were observed on JM101 cells. A complementary strand primed with the mutagenic oligonucleotide was then produced in vitro. The new strand, containing the mutation, contained thymidine and was therefore viable in JM101; the wild-type template was not.

Template DNA was prepared by PEG precipitation of the phage supernatant followed by phenol-chloroform extraction and ethanol precipitation. One $\mu$g of this template DNA was hybridized with 10 $\mu$g of oligonucleotide ZC986 by briefly boiling, incubating at 65°C for 5 minutes, and then slowly bringing the temperature down to 4°C before adding 10 $\mu$l 0.2 M HEPES pH 7.8, 2 $\mu$l 100 mM DTT, 1 $\mu$l 1 M MgCl$_2$, 20 $\mu$l 2.5 mM each dNTP, 10 $\mu$l 10 mM ATP, 1 $\mu$l 2.5 U/$\mu$l Klenow, and 2 $\mu$l 1 U/$\mu$l T$_4$ DNA ligase, final volume adjusted to 100 $\mu$l with H$_2$O. After extension at 37°C for 2 hours, the DNA was transfected into competent E. coli JM101 cells. A control extension (minus oligonucleotide) was performed to compare the amount of background produced by extension by priming on contaminating RNA or DNA species. The transfection produced zero plaques with unmutagenized template, 150 on control extension (minus oligonucleotide) and 300 with mutagenized template.

The plates were screened by hybridizing a plaque lift with $^{32}$P-labeled mutagenic oligonucleotide and washing in 3 M TMACl (Wood et al., Proc. Natl. Acad. Sci. USA 82:1585-1588, 1985) at Tm-5°C for 30 minutes and also by sequencing randomly picked plaques. One positive clone was obtained.

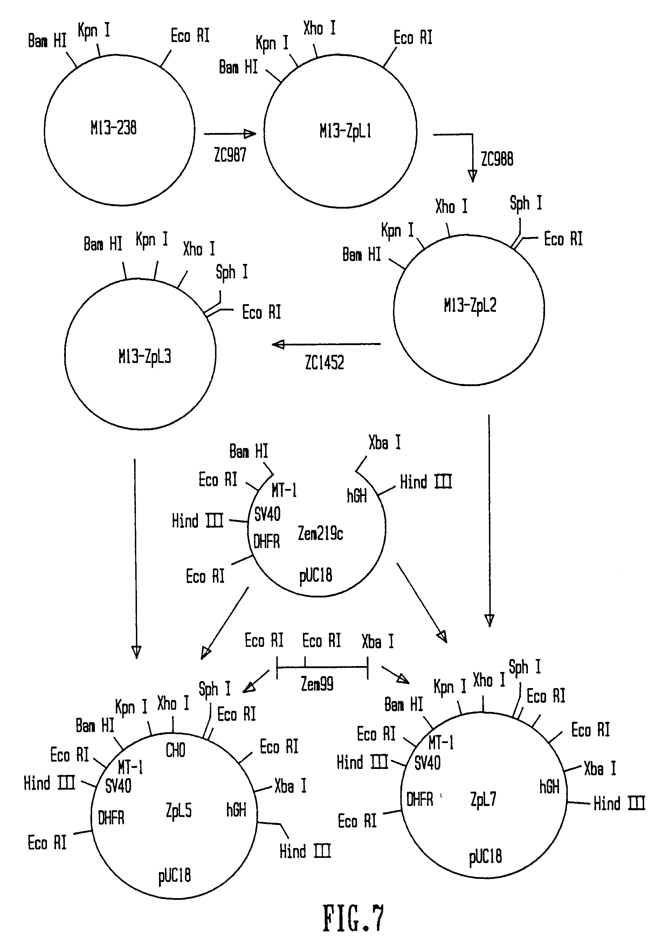

The mutagenized Bam HI Eco RI fragment containing the Kpn I site was further mutagenized to insert an Xho I site at the 3′ end of the GF domain. Template DNA was prepared from the M13 phage clone containing the Kpn I site by PEG precipitation of the phage supernatant followed by phenol-chloroform extraction and ethanol precipitation. One $\mu$g of this DNA was hybridized with 10 $\mu$g of oligonucleotide ZC987 (5′ CAC GTG GCT CGA GTA TCT ATT TC 3′). The mixture was boiled briefly, incubated at 65°C for 5 minutes, and the temperature brought slowly down to 4°C before adding 10 $\mu$l of 0.2 M HEPES pH 7.8, 2 $\mu$l of 100 mM DTT, 1 $\mu$l of 1 M MgCl$_2$, 20 $\mu$l of 2.5 mM each dNTP, 10 $\mu$l of 10 mM ATP, 2.5 units of DNA polymerase I (Klenow fragment) and 2 units of T$_4$ DNA ligase, final volume adjusted to 100 $\mu$l with H$_2$O. After extension at 37°C for 2 hours, the DNA was transfected into competent E. coli JM101 cells. The plates were screened by hybridization and after sequence confirmation of the mutagenesis, the new phage clone (ZpL1) was used to prepare template for a second mutagenesis. Oligonucleotide ZC988 (5′ CTC AGA GCA TGC AGG GG 3′) was used to introduce an Sph I site at the 3′ end of the first kringle domain. The mutagenesis was confirmed and the phage clone was designated ZpL2. Replicative form DNA was prepared from ZpL2 and the Bam HI-Eco RI fragment comprising the 5′ portion of the t-PA coding sequence was isolated. This fragment was ligated to the partial Eco RI-Xba I 3′ t-PA fragment from Zem99 and Bam HI + Xba 1 digested Zem219c (Figure 7). The resulting construct was designated ZpL7.

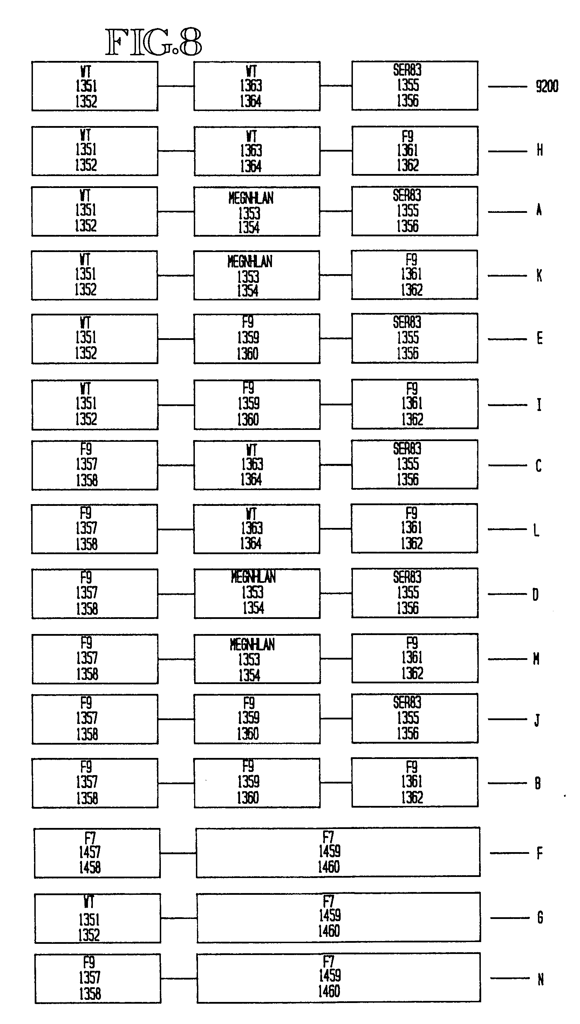

Growth factor domain replacements were constructed using the oligonucleotides shown in Table 1. The eighteen oligonucleotides are combined in various combinations of four or six sequences to generate the combinations shown in Figure 8. 500 ng of each oligonucleotide was incubated in a separate tube containing 50 mM Tris-HCl (ph 8.0), 10 mM MgCl$_2$, 150 mM dithiothreitol containing 10 $\mu$Ci (0.3 $\mu$M) $^{32}$P-ATP and 0.5 units T$_4$ polynucleotide kinase at 37°C for 30 minutes (total reaction volume - 30 $\mu$l). Five $\mu$l aliquots of reaction mixtures containing specific oligonucleotides were mixed in the combinations specified in Figure 8 (total volume of 20 $\mu$l when four oligonucleotides were mixed; 30 $\mu$l when six were mixed). One unit of T$_4$ DNA ligase was added and the mixtures were incubated at room temperature for two hours. The ligated oligonucleotide assemblies were electrophoresed on a 5% polyacrylamide gel containing 8 M urea. The bands corresponding to the full length assembled fragments were excised, eluted overnight in 0.3 M sodium acetate and the DNA was ethanol precipitated. The oligonucleotide assemblies were diluted 1-, 10- and 50-fold and added to ligation mixtures with purified Kpn I, Xho 1-cut ZpL7. Correctly substituted plasmids were identified initially by Southern blot analysis and then by sequencing across the entire substituted region. Referring to Figure 8, Fragment A replaces t-PA amino acids 63-70 with the sequence MEGNHLAN, Fragment B replaces t-PA amino acids 51-84 with the entire growth factor domain of human factor IX, Fragment C replaces t-PA amino acids 51-60 with the corresponding region of human factor IX, Fragment D replaces t-PA amino acids 51-60 with the corresponding region of factor IX and amino acids 63-70 with MEGNHLAN, Fragment E replaces t-PA amino acids 63-70 with the corresponding region of human factor IX, Fragment F replaces t-PA amino acids 51-84 with the entire growth factor region of human factor VII and Fragment G replaces t-PA amino acids 63-84 with the corresponding region of human factor VII. In fragments A, C, D and E the free cysteine at 83 is changed to serine to enhance protein stability.

The paired oligonucleotides (1351 + 1352, 1353 + 1354, 1355 + 1356, 1357 + 1358, 1359 + 1360, 1361 + 1362, 1363 + 1364, 1457 + 1458, and 1459 + 1460) may also be assembled in other combinations as shown in Figure 8.

TABLE 1

ZC1351                                    27
CTG TTA AAT CTT GTT CTG AAC CTA CAT CTT TTA ATG CAG GA

ZC1352                                    27
ATT AAA ACA TCT AGG TTC AGA ACA AGA TTT AAC AGG TAC

ZC1353                                    27
ACA TGC ATG GAA GGA AAT CAT CTT GCT AAT TTT GTT TGT CAA TGT
CCT

ZC1354                                    27
TTG ACA AAC AAA ATT AGC AAG ATG ATT TCC TTC CAT GCA TGT TCC
TCC

ZC1355                                    27
GAA GGA TTT GCT GCA AAA TCT TGT GAA ATT GAT AC

ZC1356                                    27
TCG AGT ATC AAT TTC ACA AGA TTT TCC AGC AAA TCC TTC AGG ACA

ZC1357                                    27
CTG TTA AAT CTT GTG AAT CTA ATC CTT GTC TTA ATG CAG CA

ZC1358                                    27
ATT AAG ACA AGG ATT AGA TTC ACA AGA TTT AAC AGG TAC

ZC1359                                    27
TCT TGT AAA GAT GAT ATT AAT TCA TAT GAA TGT TCG TGT CCT

ZC1360                                    27
CCA ACA TTC ATA TGA ATT AAT ATC ATC TTT ACA AGA TCC TCC

ZC1361                                    27
TTT GGA TTT GAA GGA AAA AAT TGT GAA ATT GAT AC

ZC1362                                    27
TCG AGT ATC AAT TTC ACA ATT TTT TCC TTC AAA TCC AAA AGG ACA

ZC1363                                    27
ACA TGT CAA CAA GCT CTT TAT TTT TCT CAT TTT GTT TGT CAA TGT
CCT

ZC1364                                    27
TTG ACA AAC AAA ATC AGA AAA ATA AAG AGC TTG TTG ACA TGT TCC
TCC

ZC1457                                    27
TCG GTA CCT GTT AAA TCT TGT GCT TCT TCT CCT TGT CAA AAT GGA
GGA TCT TGT AAA GAT CAA CTT CAA

EP 0 293 936 B1

ZC1458
ACC CAT GGA CAA TTT AGA ACA CGA AGA AGA GGA ACA GTT TTA CCT
CCT ACA ACA TTT CTA GTT GAA GTT

ZC1459
TCT TAC ATA TGT TTT TGT CTT CCT GCT TTT GAA GGA AGA AAT TGT
GAA ATT GAT ACT CGA GCT

ZC1460
AGA ATG TAT ACA AAA ACA GAA GGA CGA AAA CTT CCT TCT TTA ACA
CTT TAA CTA TGA GCT CGA

To construct expression vectors for the modified t-PA sequences, the vector ZpL7 was digested with Kpn 1 and Xho I and the vector fragment was gel purified. Vector DNA was ligated with each of the growth factor replacement fragments A, B, C, D, E, F, G, H, I, J and K. The ligation mixtures were used to transform competent E. coli HB101 cells. Clones were picked and DNA was prepared, digested with Eco RI and fractionated on a 1% agarose gel. The DNA was transferred to nitrocellulose and probed with the appropriate $\gamma^{32p}$-labeled oligonucleotide to confirm the presence of the substitutions. The resultant plasmids, designated ZpL7A, B, C, D, E, F, G, H, I, J and K, were digested with Eco RI and Bam HI and the 5′ t-PA fragments were cloned into M13 phage vectors and sequenced for further confirmation. The plasmids were then transfected into tk⁻BHK cells and high producing clones were scaled up for protein production and characterization.

Example 6 - Substitution for Cys in t-PA Analogs with Altered Activation Sites

For site-specific mutagenesis, a 472 bp Eco RI fragment comprising the t-PA sequence from bp 802 to bp 1274 was isolated from Zem99 and cloned into the Eco RI site of M13mp18 (replicative form). The recombinant phage were transfected into E. coli (JM101), and anti-sense strand DNA was isolated.

Site-specific mutagenesis was then carried out on the single-stranded anti-sense template DNA using one of the mutagenic primers shown in Table 2 and ZC87 as second primer. Oligonucleotides ZC487, 488, 489 and 620 change the Phe at position 274 to Glu, Gly, Arg or Pro, respectively. Oligonucleotides ZC797, 874, 1013 and 1027 change the Arg at position 275 to Gly, Leu, Pro or Asp, respectively. Oligonucleotide 621 introduces a Leu in place of the Lys at position 277. Oligonucleotide 928 changes the Ile at position 276 to Pro. Oligonucleotide 875 changes Arg (275) to Leu and oligonucleotide 927 changes Phe (274) to Pro in the mutant which previously had Lys (277) converted to Leu. Thus, oligonucleotides 875 and 927 can be used to generate double mutations. Twenty pmoles of phosphorylated mutagenic primer and 20 pmoles of the second primer were combined with one pmole of single-stranded template in 10 µl of 20 mM Tris pH 7.5 , 10 mM MgCl₂, 50 mM NaCl, 1 mM DTT and incubated at 65°C for 10 minutes, then 5 minutes at room temperature, and placed on ice. Ten µl of 20 mM Tris pH 7.5, 10 mM MgCl₂, 2 mM ATP, 10 mM DTT containing 1 mM dNTPs, 2.5 units Klenow polymerase, and 3.5 units DNA ligase were added to the annealed DNA, and the mixture was incubated 3 hours at 15°C. The DNA was then transfected into competent E. coli JM101, and the cells were plated on YT agar and incubated at 37°C. The DNA was then transferred to nitrocellulose and prehybridized at the Tm-4°C of the mutagenic primer for 1 hour in 6x SSC, 10x Denhardt's and hybridized to ³²P-labeled mutagenic primer at Tm-4°C in the same solution. After three washes at Tm-4°C, filters were exposed to X-ray film overnight. Additional wash steps were performed at 5°C higher increments as necessary to identify mutant plaques. The mutated inserts were sequenced by the dideoxy method.

15

TABLE 2

ZC463 5'CCT CAG TTT AAA ATC AAA3'

ZC487 5'CAG CCT CAG GAG CGC ATC AAA3'

ZC488 5'CAG CCT CAA GGT CGC ATC AAA3'

ZC489 5'CAG CCT CAG AGA CGC ATC AAA3'

ZC620 5'CAG CCT CAG CCT CGC ATC AA3'

ZC621 5'TTT CGC ATC CTC GGA GGG CTC3'

ZC797 5'CTT CAG TTC GGC ATC AAA3'

ZC814 5'G CCT CAG TTC GGC ATC AAA GC3'

ZC874 5'CT CAG TTT CTC ATC AAA GG3'

ZC875 5'CT CAG TTT CTC ATC CTC GG3'

ZC927 5'CAG CCT CAG CCT CCC ATC CT3'

ZC928 5'CAG TTT CGC CCC AAA GGA GG3'

ZC1013 5'CT CAG TTT CCC ATC AAA GC3'

ZC1027 5'CCT CAG TTT GAC ATC AAA GG3'

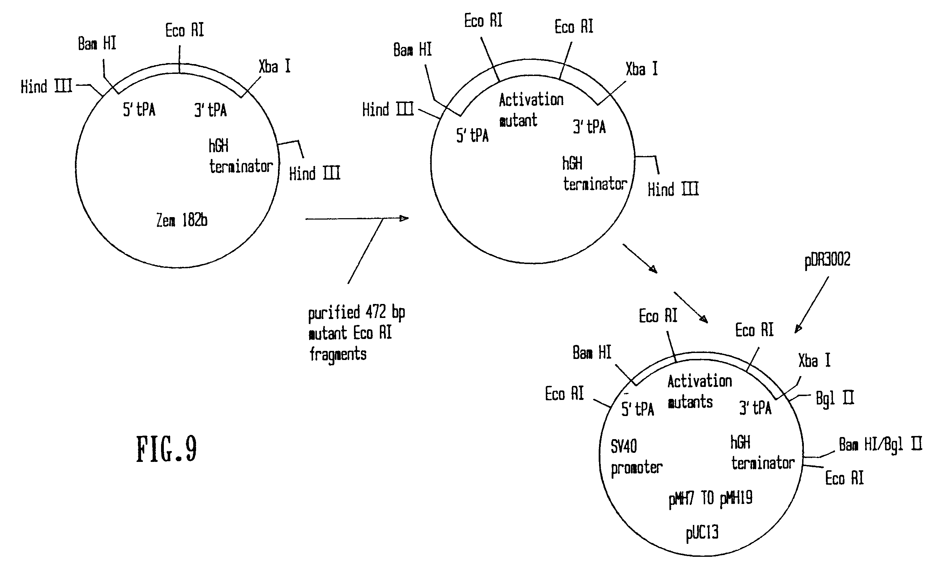

Expression vectors for the altered sequences were then constructed (Figure 9). Replicative form (RF) DNA was prepared from the mutagenized phage, and the modified t-PA sequences were purified as Eco RI fragments. Plasmid Zem182b was digested with Eco RI, the vector sequences containing the 5′ and 3′ portions of the t-PA coding sequence were treated with calf alkaline phosphatase, and the modified t-PA sequences were inserted. The resultant plasmids were digested with Bam HI and Xba I, and the t-PA fragments were inserted into Bam HI + Xba I - cut pDR3002. The resultant vectors were designated pMH7 through pMH20 (Table 3).

16

## TABLE 3

| Protein | Sequence of Amino Acids 273-279 |
|---|---|
| Native t-PA | Gln-Phe-Arg-Ile-Lys-Gly-Gly |
| pMH7 | Gln-Gly-Arg-Ile-Lys-Gly-Gly |
| pMH8 | Gln-Phe-Arg-Ile-Leu-Gly-Gly |
| pMH9 | Gln-Arg-Arg-Ile-Lys-Gly-Gly |
| pMH10 | Gln-Pro-Arg-Ile-Lys-Gly-Gly |
| pMH11 | Gln-Glu-Arg-Ile-Lys-Gly-Gly |
| pMH12 | Gln-Phe-Lys-Ile-Lys-Gly-Gly |
| pMH13 | Gln-Phe-Gly-Ile-Lys-Gly-Gly |
| pMH14 | Gln-Pro-Arg-Ile-Leu-Gly-Gly |
| pMH15 | Gln-Phe-Leu-Ile-Lys-Gly-Gly |
| pMH16 | Gln-Phe-Leu-Ile-Leu-Gly-Gly |
| pMH17 | Gln-Phe-Arg-Pro-Lys-Gly-Gly |
| pMH18 | Gln-Phe-Pro-Ile-Lys-Gly-Gly |
| pMH19 | Gln-Phe-Asp-Ile-Lys-Gly-Gly |
| pMH20 | Gln-Phe-Gly-Ile-Leu-Gly-Gly |

The modified GF domain and activation site mutations are then combined in the following manner. Cys (84) mutant DNA from Zem99-9200 is isolated as a Bam HI-Sca I fragment and joined, in a three-part ligation, to Bam HI, Xba I-digested Zem219b and the Sca I-Xba I fragment encoding the modified activation site, from pMH10, pMH13 or pMH17. The resultant expression vectors, designated 9200-10, 9200-13 and 9200-17, are used to transfect tk⁻BHK cells by electroporation. High producer clones are scaled up and the protein is purified.

Example 7 - Substitution for Cys in a t-PA Analog Containing a Consensus Finger Domain

Replacement of the t-PA finger domain with a consensus finger region results in the elimination of potential proteolytic cleavage sites at Arg-27, Lys-49 and Arg-89. Eight finger replacement sequences were constructed, based on an analysis of the finger domains of fibronectin and t-PA.

The consensus finger sequences were constructed from oligonucleotides as described below, then inserted into the t-PA coding sequence. To facilitate this insertion, a Kpn I site was introduced downstream (3′) of the region encoding the wild-type finger domain. Digestion of the resulting sequence with Bgl II and Kpn I resulted in the deletion of the wild-type finger domain.

A. Kpn I Site Insertion Between the Finger and Growth Factor Domains

In order to place a Kpn I site after the finger domain in t-PA, a mutagenesis was performed with oligonucleotide ZC986 (5′ TTT GAC AGG TAC CGA GTG CCA 3′). DNA of a phage M13 clone containing the 5′ Bam HI-Eco RI fragment of the native t-PA cDNA was prepared. 100 μl of the DNA solution was used to infect E. coli RZ1032 in 100 μl of YT medium supplemented with 0.1 μg/ml uridine. This culture was incubated at 37°C, with vigorous shaking, overnight. Growing the M13 in RZ1032 produces phage containing uridine which are viable in RZ1032 but not in JM101.

The cells were spun out, and the phage supernatant was used to reinfect E. coli RZ1032. This second passage was performed to dilute out any JM101-derived phage which contained no uracil. Again, the cells

17

were spun out and the phage were plated on JM101 and RZ1032. Normal viability was observed on RZ1032 plates (indicating phage at $10^9$ pfu/ml), but no plaques were observed on JM101 cells. A complementary strand primed with the mutagenic oligonucleotide was then produced in vitro. The new strand, containing the mutation, contained thymidine and was therefore viable in JM101; the wild-type template was not.

Template DNA was prepared by PEG precipitation of the phage supernatant followed by phenol-chloroform extraction and ethanol precipitation. One $\mu$g of this template DNA was hybridized with 10 $\mu$g of oligonucleotide ZC986 by briefly boiling, incubating at 65°C for 5 minutes, and then slowly bringing the temperature down to 4°C before adding 10 $\mu$l of 0.2 M HEPES pH 7.8, 2 $\mu$l 100 mM DTT, 1 $\mu$l 1 M MgCl$_2$, 20 $\mu$l 2.5 mM each dNTP, 10 $\mu$l 10 mM ATP, 1 $\mu$l 25 U/$\mu$l Klenow, and 2 $\mu$l 1 U/$\mu$l T$_4$ DNA ligase, final volume adjusted to 100 $\mu$l with H$_2$O. After extension at 37°C for 2 hours, the DNA was transfected into competent JM101 cells. A control extension (minus oligonucleotide) was performed to compare the amount of background produced by extension by priming on contaminating RNA or DNA species. The transfection produced zero plaques with unmutagenized template, 150 on control extension (minus oligonucleotide) and 300 with mutagenized template.

The plates were screened by hybridizing a plaque lift with [32P]-labeled mutagenic oligonucleotide and washing in 3 M TMACl (Wood et al., Proc. Natl. Acad. Sci. USA 82: 1585-1588, 1985) at Tm-5°C for 30 minutes and also by sequencing randomly picked plaques. One positive clone was obtained.

B. Production of Finger Replacement Domains

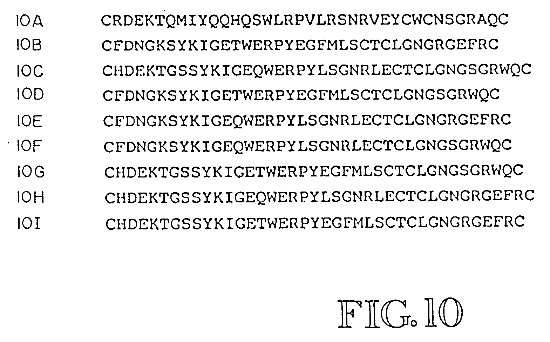

The consensus finger region replacements shown in Table 4 and Figure 10 were constructed.

## TABLE 4

Finger        Encoded Amino Acid Sequence    Oligonucleotides[A]

t-PA wild-type:
    CRDEKTQMIYQQHQSWLRPVLR-SNRVEYCWC--N-SGRAQC

Consensus 1:                                  (ABC)
    CFD--NGKSYKTGETWERPYE--GFMLS-CTCLGNGRGEFRC

Consensus 2:                                  (DEF)
    CHDEKTGSSYKIGEQWERPYL-SGNRLE-CTCLGNGSGRWQC

Consensus 3:                                  (ABF)
    CFD--NGKSYKIGETWERPYE--GFMLS-CTCLGNGSGRWQC

Consensus 4:                                  (AEC)
    CFD--NGKSYKIGEQWERPYL-SGNRLE-CTCLGNGRGEFRC

Consensus 5:                                  (AEF)
    CFD--NGKSYKIGEQWERPYL-SGNRLE-CTCLGNGSGRWQC

Consensus 6:                                  (DBF)
    CHDEKTGSSYKIGETWERPYE--GFMLS-CTCLGNGSGRWQC

Consensus 7:                                  (DEC)
    CHDEKTGSSYKIGEQWERPYL-SGNRLE-CTCLGNGRGEFRC

Consensus 8:                                  (DBC)
    CHDEKTGSSYKIGETWERPYE--GFMLS-CTCLGNGRGEFRC

*A = ZC1116/1117            D = ZC1122/1123
 B = ZC1118/1119            E = ZC1124/1125
 C = ZC1120/1121            F = ZC1126/1127

# TABLE 5

ZC1116
GAT CTT ATC AAG TCA TAT GTT TTG ATA ATG CAA AAT CTT ATA A

ZC1117
CTC CAA TTT TAT AAG ATT TTC CAT TAT CAA AAC ATA TGA CTT GAT
AA

ZC1118
AAT TGG AGA AAC ATG GGA ACG GCC GTA TGA AGG ATT TAT CCT TTC
T

ZC1119
CAT GTA CAA GAA AGC ATA AAT CCT TCA TAC GGC CGT TCC CAT GTT
T

ZC1120
TGT ACA TCC CTA GGA AAT CGC CGC GGA GAA TTT AGA TGT CAT TCC
GTA C

ZC1121
CGA ATG ACA TCT AAA TTC TCC GCG GCC ATT TCC TAG G

ZC1122
GAT CTT ATC AAG TCA TAT GTC ATG ATG AAA AAA CAG GCT CGA GTT
ATA A

ZC1123
CTC CAA TTT TAT AAC TCG AGC CTG TTT TTT CAT CAT GAC ATA TGA
CTT GAT AA

ZC1124
AAT TGG AGA ACA ATG GGA ACG GCC GTA TCT TTC TGG AAA TCG ATT
AGA A

ZC1125
CAT GTA CAT TCT AAT CGA TTT CCA GAA AGA TAC GGC CGT TCC CAT
TGT T

ZC1126
TGT ACA TGC CTA GGA AAT GGT TCC GGA AGA TGG CAA TGT CAT TCG
GTA C

ZC1127
CGA ATG ACA TTG CCA TCT TCC GGA ACC ATT TCC TAG G

    The eight consensus sequences were generated from the indicated oligonucleotides. The oligonucleotides (Table 5) were produced using an Applied Biosystems Model 380A DNA synthesizer. First, the twelve oligonucleotides were kinased and simultaneously labeled to a low specific activity with $\gamma$-$^{32P}$ ATP by incubating each with polynucleotide kinase at 37°C for ½ hour. Then the indicated eight combinations (ABC, DEF, ABF, AEC, AEF, DBF, DEC and DBC) were produced by mixing the appropriate oligonucleotides, adding DNA ligase, and incubating at 37°C for 1 hour. The products of this reaction were sorted out on a 6% polyacrylamide-8 M urea sequencing gel. The bands corresponding to the DNA coding for full-length finger domains were cut out, and the DNA was eluted in 2.5 M ammonium acetate. The DNA was ethanol-precipitated and resuspended in water to a concentration of 1 pmole/µl.

RF DNA was prepared from the positive clone described in Example 5A, and the Bam HI to Eco RI t-PA fragment was purified. Plasmid Zem219a (described in Example 2) was digested with Xba I and then partially digested with Eco RI. The 1010 bp fragment, containing the 3′ t-PA coding region, was purified. Plasmid Zem219b (described in Example 2) was digested with Bam HI and Xba I and ligated to the 5′ t-PA fragment (Bam HI-Eco RI) and the 1010 bp Eco RI-Xba I fragment. The resulting vector, designated Zem238, contains a Kpn I site after the finger domain. Zem238 was digested with Bgl II and Kpn I, gel-purified to remove the wild-type finger domain, and ligated with each of the eight consensus sequences to generate expression vectors 238-Fcon 1 to 238-Fcon 8.

C. Combination of Cys (84) Substitution with Consensus Finger

Template DNA from M13-9200 (Example 4) is annealed to oligonucleotide primer ZC986, as described in Example 7A. A correctly mutated clone with a Kpn I site inserted at the 3′ end of the finger domain is identified and sequenced. RF DNA from this clone is digested with Kpn 1 and Xba I, and the Cys (84) mutant DNA is ligated to vector DNA from Kpn I, Xba 1-digested plasmids 238-Fcon 1 to 238-Fcon 8. The resultant vectors, designated 9200-Fcon 1 to 9200-Fcon 8, are transfected into tk⁻BHK cells and the mutant proteins are purified and characterized.

Example 8 - Modification of Carbohydrate Addition Sites in t-PA Analogs with Alterations in the GF Domain

Tissue plasminogen activator contains four potential glycosylation sites (amino acid sequence Asn-X-Ser/Thr. According to Pohl et al. (Biochemistry 23: 3701-3707, 1984), three of these sites (Asn-117, -184 and -448) are glycosylated in t-PA obtained from Bowes melanoma cells. By altering the Asn residues at the glycosylation sites to other amino acid residues, the glycosylation is blocked. It is particularly preferred that the Asn residues be changed to Gln residues. Alterations in the DNA sequence encoding t-PA are made by site-specific mutagenesis. Nucleotide changes may be made singly or in combination to alter one or more of the glycosylation sites. Additionally, mutated sequences may be combined with DNA fragments comprising other deletions, insertions or substitutions of the t-PA coding sequence in order to generate new proteins comprising several mutations in combination.

Site-specific mutagenesis was carried out on single-stranded M13 phage templates using mutagenic primers ZC294, ZC295 and ZC297 (see Table 6) to produce the alterations at Asn-117, -184 and -448, respectively. DNA sequences encoding mutant t-PAs were then constructed comprising single, double or triple mutations by replacing the t-PA coding sequences in pDR1296. The following plasmids were constructed: (1) pDR1601, comprising the mutation at Asn-117; (2) pDR1602, comprising the mutation at Asn-184; (3) pDR1603, comprising the mutation at Asn-448; (4) pDR1604, comprising the mutations at Asn-117 and -448; (5) pDR1605, comprising the mutations at Asn-184 and -448; and (6) pDR1606, comprising the three mutations.

## TABLE 6

| Primer | Sequence |
|---|---|
| ZC294 | 5′ACC AAC TGG CAA TCT TCT GCG TTG GCC3′ |
| ZC295 | 5′TGC TAC TTT GGT CAA GGG TCA GCC3′ |
| ZC297 | 5′CAA CAT TTA TTG CAA AGA ACA GTC3′ |

In an additional mutagenesis, an oligonucleotide primer (5′ ACG GTA GGC TGT CCC ATT GCT AAA GTA GCA 3′) was prepared in order to replace Gly (183) and Ser (186) with Ser and Thr, respectively. These mutations result in more uniform glycosylation of the K2 domain. Site-specific mutagenesis was performed according to the one-primer method, using the template M13mp18/Bam-Zem99 (Example 3). Single-stranded mutated phage DNA was prepared and sequenced. A clone having the desired sequence alteration was designated M13-6000.

RF DNA from M13-600 was isolated, digested with Bgl II and Apa I, and a fragment of approximately 1.4 kb was isolated. This fragment was joined to Bgl II, Apa I-digested Zem99 to produce the vector Zem99-6000. E. coli RR1 transformed with Zem99-6000 has been deposited with the Fermentation Research Institute under Accession No. FERM P-9126.

Cys (84) mutant DNA from plasmid Zem99-9200 is digested with Bgl II and Nar I. The mutant DNA sequences are isolated from pDR1601 and Zem99-6000 as Nar I-Xba I fragments. Plasmid 9200-1601 is generated by ligation of the Bgl II-Nar I fragment from Zem99-9200, the Nar 1-Xba 1 fragment from pDR1601 and the Bgl II-Xba I (vector) fragment of Zem219a. Plasmid 9200-6000 is generated by ligation of the Bgl II-Nar I fragment from Zem99-9200, the Nar I-Xba I fragment from Zem99-6000, and the Zem219a vector fragment. These plasmids are used to transfect tk⁻BHK cells by electroporation. The mutant proteins are purified and characterized.

The mutant t-PA sequences of pDR1601 and pDR1606 were isolated as Bam HI-Xba I fragments and inserted into Zem99. The resulting expression vectors were transfected into tk⁻ BHK cells. Mutant proteins 1601 and 1606 were isolated from the transfected cells and characterized.

To replace the Asn in the K1 glycosylation site of the GF replacement mutants described in Example 5, template DNA was prepared from phage ZpL2 and mutagenized with oligonucleotide ZC1452 (5′ CAA CGC GCT AGA TTG CCA GTT GGT 3′). The resultant phage clone was designated ZpL3 (Figure 7).

Replicative form DNA was prepared from ZpL3 and the Bam HI-Eco RI fragment comprising the 5′ portion of the t-PA coding sequence was isolated. This fragment was ligated to the partial Eco RI-Xba I 3′ t-PA fragment from Zem99 and Bam HI + Xba I digested Zem219c (Figure 7). The resultant vector, designated ZpL5, was digested with Kpn I and Xho I and the vector fragment was gel purified. Vector DNA was ligated with the growth factor replacement fragments A, B, D, and G described in Example 5. The ligation mixtures were used to transform competent E. coli HB101 cells. Clones were picked and the GF domain replacements were confirmed by hybridization and sequence analysis for plasmids ZpL5A, B and D. ZpL5A was transfected into tk⁻BHK cells and high producing clones were scaled up for protein production and characterization.

Example 9 - Combination of GF Modifications with Kringle Domain Replacement

A. Asp (96) Plasminogen Kringle

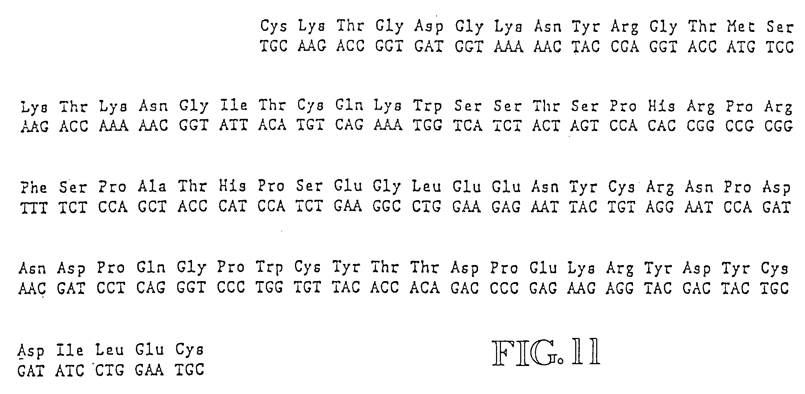

Plasmid pK1 comprises a coding sequence for the K1 domain of plasminogen, the sequence of which is shown in Figure 11. It was constructed from a series of eleven oligonucleotides designated PK1-1, PK1-2, PK1-3, --PK1-12, the sequences of which are shown in Table 7.

22

TABLE 7

| Oligonucleotide | Sequence |
|---|---|
| PK1-1 | 5'GAT CCA CGC GTG CCA CGT GCA AGA CCG GTG ATG GTA AAA ACT ACC GAG GTA CCA TGT CCA AGA CC3' |
| PK1-2 | 5'AAA AAC GGT ATT ACA TGT CAG AAA TGG TCA TCT ACT AGT CCA CAC CGC CCG CGG TTT TCT3' |
| PK1-3 | 5'CCA GCT ACC CAT CCA TCT GAA GCC CTG GAA GAG AAT TAC TGT AGG AAT CCA GAT AAC GAT3' |
| PK1-4 | 5'CCT CAG GGT CCC TGG TGT TAC ACC ACA GAC CCC GAG AAG AGG TAC GAC TAC TGC GAT ATC GCA TG3' |
| PK1-5 | 5'CCG TTT TTG GTC TTG G3' |
| PK1-6 | 5'GTA GCT GGA GAA AAC CG3' |
| PK1-7 | 5'CCC TGA GGA TCG TTA TC3' |
| PKl-9 | 5'CGA TAT CGC AGT AGT CGT ACC TCT TCT C3' |
| PK1-10 | 5'GAT CCT CAG GGT CCC TGG TGT TAC ACC ACA3' |
| PK1-11 | 5'GAC CCC GAG AAG AGG TAC GAC TAC TGC GAT ATC GCA TG3' |
| PK1-12 | 5'GGG GTC TGT GGT GTA ACA CCA GGG ACC CTG AG3' |

The coding sequence for nucleotides 1 through 182 of the plasminogen K1 domain was constructed from oligonucleotides PK1-1 through PK1-7 in the following manner. 100 pmole each of the oligonucleotides PK1-1, PK1-2, PK1-3 and PK1-4 were phosphorylated at their 5' termini. The phosphorylated oligonucleotides were mixed with 100 pmole each of PK1-5, PK1-6, and PK1-7. The mixture was precipitated with ethanol, and the precipitate was resuspended in $H_2O$ and heated for 3 minutes at 90°C. The solution was then left to stand at room temperature for ten minutes, then placed on ice. To the chilled mixture was added 10 $\mu$l of 660 mM Tris HCl, pH 7.6, containing 6.6 mM $MgCl_2$, 10 $\mu$l of 0.1 M dithiothreitol, 10 $\mu$l of 5 mM ATP, and 1000 units of $T_4$ DNA ligase. The mixture was incubated 15 hours at 14°C. Ethanol was added and the precipitate was resuspended in 20 $\mu$l of TE buffer (10 mM Tris-HCl, 1 mM EDTA, pH 8.0), followed by the addition of an equal volume of alkali loading buffer (20 mM NaCl, 2 mM EDTA, 80% formamide, 0.1% xylene cyanol and 0.1% bromophenol blue). The mixture was heated for three minutes at 90°C and electrophoresed on a 6% polycarylamide gel containing 8.4 M urea for one hour at 300 volts. The gel was stained with ethidium bromide and a 250 bp band was recovered by electrophoretic transfer to DEAE-cellulose paper (Dretzen et al., Anal. Biochem. 112:295-298, 1981). The recovered DNA was solubilized in 100 $\mu$l of TE buffer and the fragment was designated PK1-n. PK1-n was C-tailed at the 3' terminus by combining 10 $\mu$l of PK1-n with 2 $\mu$l of 100 mM sodium cacodylate--25 mM HEPES, pH 7.6, 6.2 $\mu$l of 1 mM dCTP, 10 units terminal deoxynucleotidyl transferase and 5 $\mu$l of $H_2O$. The reaction mix was incubated at 37°C for 10 minutes, then extracted with phenol:chloroform (1:1).

One $\mu$l of 3'-oligo (dG) tailed pUC9 (obtained from Pharmacia) was cleaved with Sma I. The linearized, tailed plasmid was added to the C-tailed PK1-n. The mixture was then ethanol precipitated and the DNA was resuspended in 0.5 $\mu$l of 2M KCl and 9.5 $\mu$l of TE buffer, and incubated at 65°C for 10 minutes, then cooled to room temperature. To the cooled mixture were added 5 $\mu$l of 0.2 M Tris HCl, pH 7.5, containing 0.1 M $MgCl_2$ and 0.1 M dithiothreitol, 20 $\mu$l of 2.5 mM DNTTs, 10 $\mu$l of 5 mM ATP, 53 $\mu$l $H_2O$, 5 units DNA

polymerase I (Klenow fragment), and 300 units T$_4$ DNA ligase (final volume of 100 $\mu$l). The mixture was incubated at 14°C for 12 hours, then used to transfect E. coli JM83.

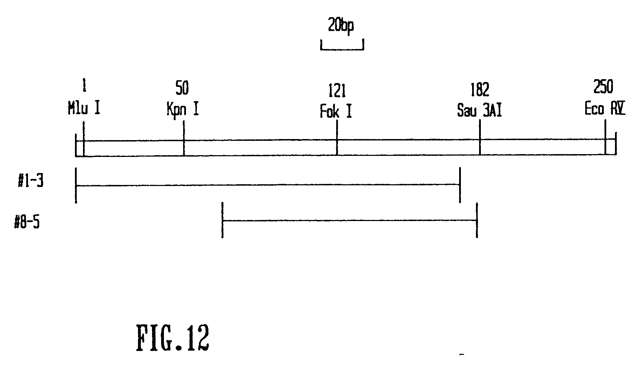

The transfected JM83 cells were probed with PK1-6 using the method of Wallace et al. (Nuc. Acids Res. 9:879-894, 1981). Twenty positive clones were sequenced and two were selected: #1-3, including base pairs 1-170, and #8-5, including base pairs 68-186 (see Figure 12).

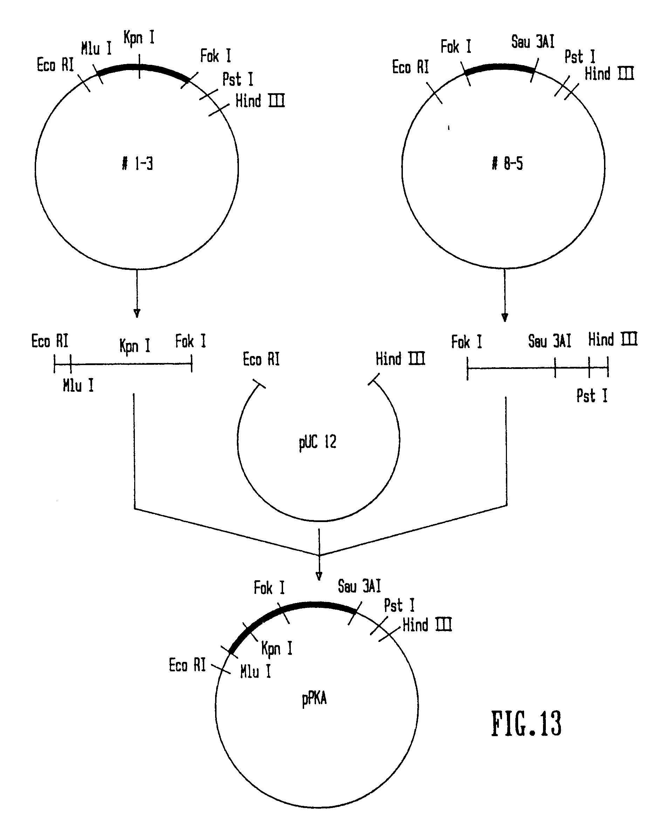

Referring to Figure 13, clone #1-3 was digested with Eco RI and Fok I, and a 130 bp fragment containing a Kpn I site was recovered. Similarly, clone #8-5 was digested with Fok I and Hind III, and a 90 bp fragment was recovered. The two fragments were joined to Eco RI, Hind III-digested pUC12 and the resultant plasmid was designated pPKA. This plasmid thus contains a DNA sequence corresponding to nucleotides 1-182 of the plasminogen K1 sequence.

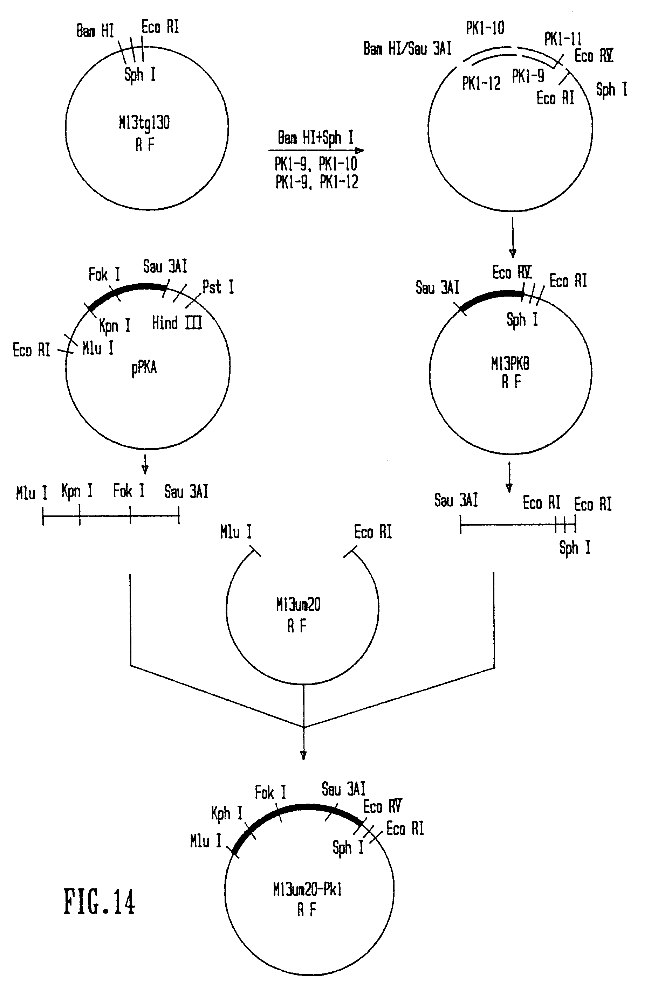

The remainder of the K1 sequence was constructed using oligonucleotides PK1-9, PK1-10, PK1-11 and PK1-12. One pmole each of the oligonucleotides was phosphorylated at the 5′ end, and the combined oligos were mixed with 40 $\mu$g of Bam HI, Sph I-digested M13tg130RF (obtained from Amersham). To this mixture were added 4 $\mu$l of 660 mM Tris-HCl, ph 7.6, containing 66 mM MgCl$_2$, and 22 $\mu$l of H$_2$O. The solution was heated for three minutes at 90°C and allowed to cool to room temperature over a period of one hour. Four $\mu$l of 0.1 M dithiothreitol, 4 $\mu$l of 5 mM ATP, and 300 units of T$_4$ DNA ligase were added, and the mixture was incubated for 12 hours at 14°C. The resulting phage clone, designated M13PKB RF (Figure 14), contained nucleotides 183 through 250 of the plasminogen K1 sequence.

The assembly of the complete plasminogen K1 coding sequence is illustrated in Figure 14. Plasmid pPKA was digested with Mlu I and Sau 3A1, and a 176 bp fragment was recovered. M13PKB RF was digested with Sau 3A1 and Eco RI, and an 88 bp fragment was recovered. These fragments were joined to Mlu I, Eco RI-digested M13um20 RF (obtained from IBI), and the resultant plasmid was designated M13um20-PK1.

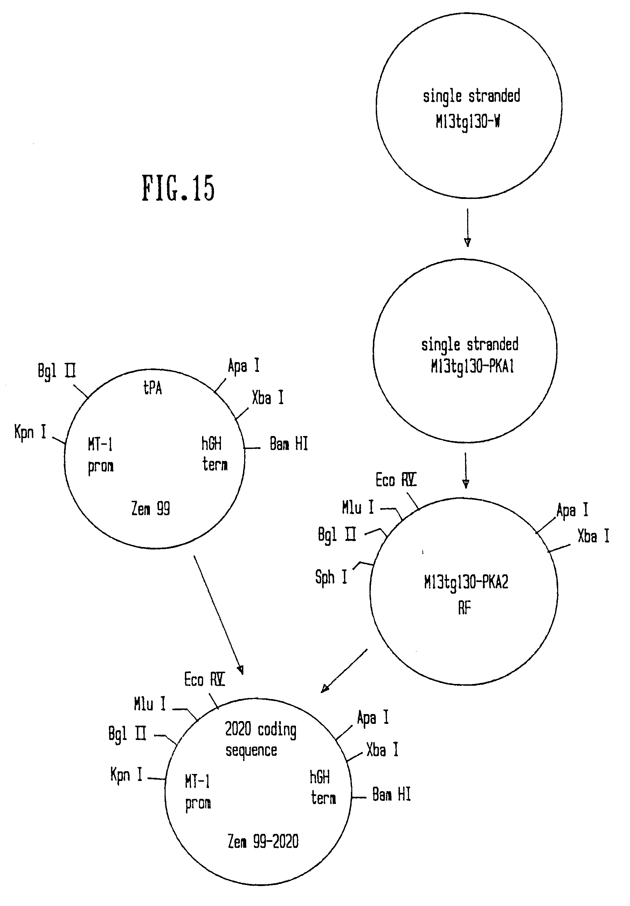

The PK1 coding sequence was then inserted into the t-PA cDNA as a replacement for the t-PA Kringle 1 sequence (Figures 15 to 16). The t-PA sequenced was first mutagenized to insert Mlu 1 and Eco RV sites. Plasmid pDR1496 was digested with Sph 1 and Xba I, and the 2.1 kb fragment comprising the alpha factor and t-PA sequences was isolated. (S. cerevisiae strain E8-11C transformed with pDR1496 has been deposited with American Type Culture Collection under Accession No. 20728.) This fragment was joined to Sph I, Xba I-digested M13tg130 (RF), and the resultant phage was designated M13tg130-W. Single-stranded phage DNA was then annealed to an oligonucleotide (5′ GCA CGT GGC ACG CGT ATC TAT TTC 3′) and mutagenesis was carried out according to standard procedures. The mutagenized phage was designated M13tg130-PKA1. Single-stranded DNA of M13tg130-PKA was isolated and mutagenized by the one-primer method with an oligonucleotide having the sequence 5′ CTC AGA GCA TTC CAG GAT ATC GCA GAA CTC 3′. Single-stranded DNA was prepared from the mutagenized phage and sequenced. A clone containing an Mlu I site at the 5′ end and an Eco RV site at the 3′ end of the Kringle 1 coding sequence was selected and designated M13tg130-PKA2.

Replicative form DNA was prepared from M13tg130-PKA2 and was digested with Bgl II and Apa I. The fragment containing the Mlu I and Eco RV sites was recovered and joined to Bgl II, Apa I-digested Zem99, as shown in Figure 15. The resultant plasmid was designated Zem99-2020.

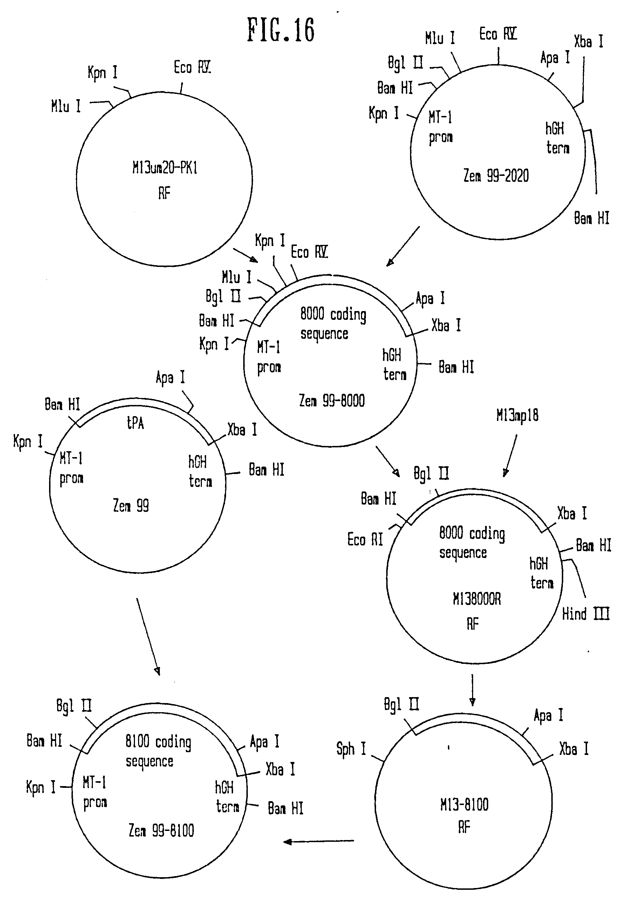

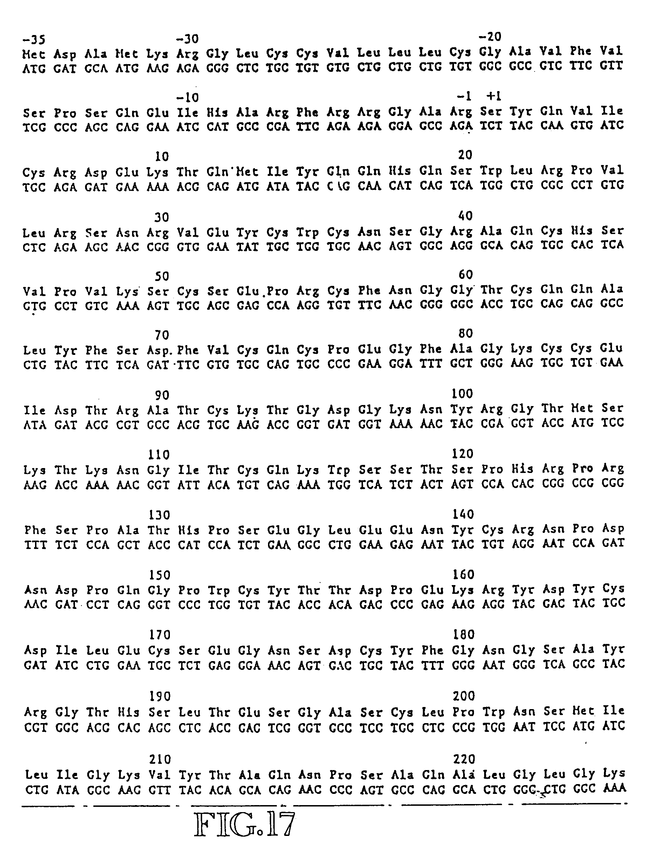

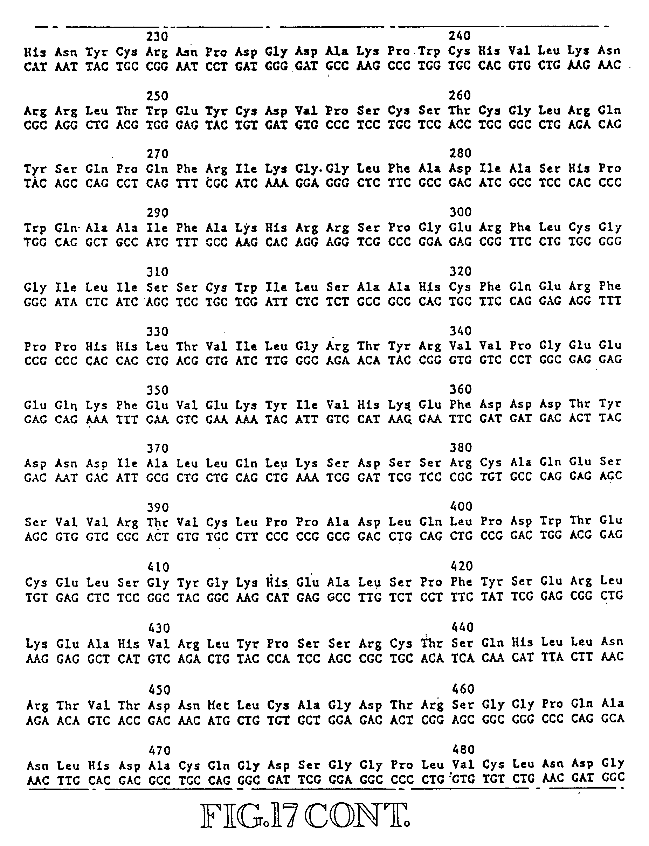

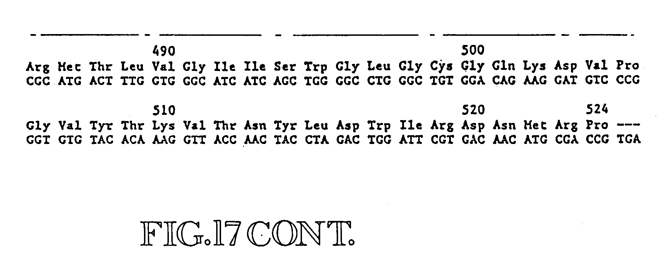

The PK1 sequence was then inserted into the t-PA cDNA. M13um20-PK1 RF was digested with Mlu I and Eco RV, and the 336 bp fragment was recovered. This fragment was joined to Mlu I, Eco RV-digested Zem99-2020 to construct Zem99-8000 (Figure 16). The t-PA coding sequence of Zem99-8000 and the encoded amino acid sequence are shown in Figure 17.

### B. Asn (96) Plasminogen Kringle

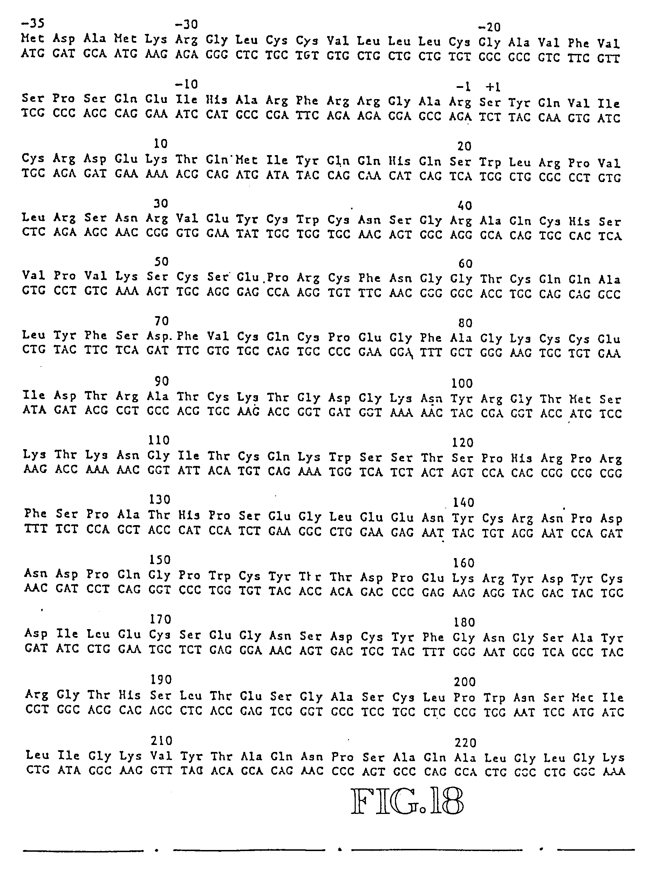

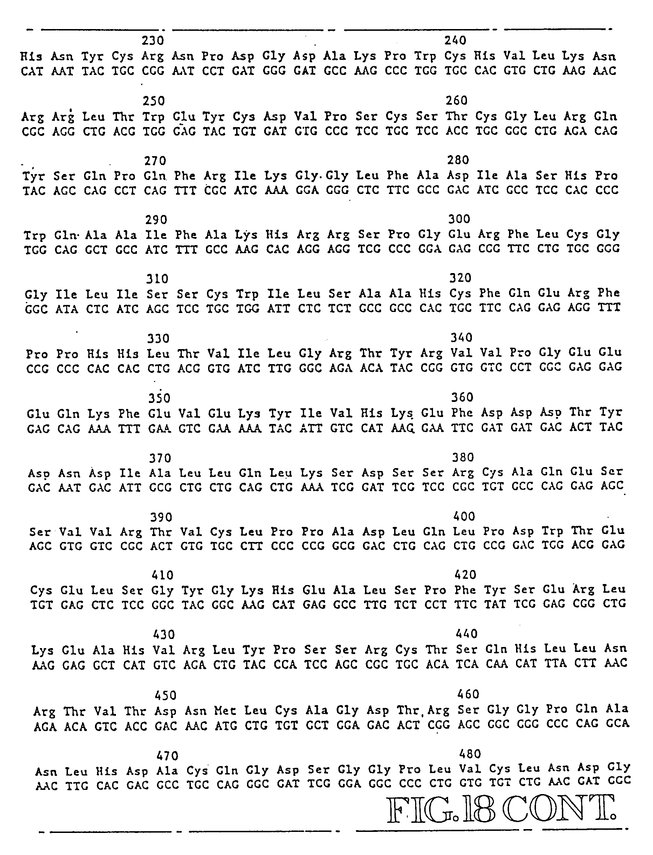

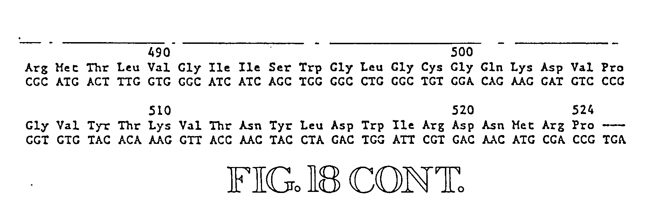

A second plasminogen K1 sequence encoding Asn at position 96 was constructed (Figure 16). Zem99-8000 was digested with Bam HI, and the fragment containing the Bgl II site was recovered. This fragment was joined to Bam HI cut M13mp18 to construct M13-8000R. An oligonucleotide primer (sequence 5′ TTT TTA CCA TTA CCG GTC TT 3′) was annealed to single-stranded M13-8000R, and mutagenesis was carried out according to routine procedures for the one-primer method. Clones were screened and sequenced, and double-stranded DNA, designated M13-8000RF, was prepared from a positive clone. This phage was digested with Bgl II and Apa I, and the t-PA fragment was isolated and joined to Bgl II, Apa I - cut Zem99. The resultant plasmid was designated Zem99-8100. The t-PA coding sequence present in Zem99-8100 and the encoded amino acid sequence are shown in Figure 18.

Plasmids Zem99-8000 and Zem99-8100 have been deposited (as E. coli RRI transformants) with FRI under Accession Nos. FERM P-9272 and FERM P-9315, respectively.

### C. Combination of GF modifications and K1 Substitution

Single-stranded DNA was isolated from M13-9200 and was mutagenized using the oligonucleotide 5′ CCA CGT GGC ACG CGT ATC TAT TTC 3′ to introduce an Mlu I site. The mutagenized phage was designated M13-92.05PKA1. RF DNA was prepared from the mutant phage and was digested with Bgl II and Mlu I, and the 264 bp fragment was recovered. Zem99-8000 was digested with Mlu I and Apa I, and the 1126 bp fragment was recovered. These two fragments were joined to Bgl II, Apa I-digested Zem99, and the resultant plasmid was designated Zem99-9280. This plasmid thus encodes a mutant t-PA with Ser at amino acid 84 and the K1 domain of plasminogen with Asp at position 96.

A second vector, which contains a mutant t-PA sequence encoding a t-PA analog with Ser at amino acid 84 and the K1 domain of plasminogen with Asn at position 96, was constructed. RF DNA from M13-92.05PKA1 was digested with Bgl II and Mlu I, and the 264 bp fragment was recovered. Zem99-8100 was digested with Mlu I and Apa I, and the 1126 bp fragment was recovered. These two fragments were joined to Bgl II, Apa I-digested Zem 99, and the resultant plasmid was designated Zem99-9281.

Plasmid Zem99-8000 was digested with Spe I and Hind III and the fragment containing the 3′ mutant t-PA coding sequence and the entire hGH terminator was gel purified. Plasmid ZpL7 was partially digested with Hind III and completely digested with Xho I and the vector sequences were purified away from the 3′ t-PA coding sequence and the hGH terminator. Oligonucleotides ZC1639 and ZC1640 (Table 3) were annealed and the resulting Xho I to Spe I fragment (encoding the 5′ portion of the plasminogen K1 kringle) was combined in a ligation reaction with the above two purified fragments. The resulting plasmid was designated 8900. To construct chimeric sequences encoding t-PA derivatives comprising the GF and K1 substitutions, plasmid 8900 is digested with Bam HI and Xho I and the GF region is replaced with the Bam HI to Xho I region isolated from one of plasmids ZpL7A-N.

### Example 10 - Characterization of Proteins

Proteins 9100 and 9200 were prepared as described and tested for activity and plasma half-life using native t-PA prepared from transfected tk⁻BHK cells as a control.

t-PA analogs #9100 and #9200 were tested for clot lysis activity using native recombinant t-PA as a control. A silk thread 3 cm in length was introduced into an Atom venous catheter (4Fr 3.5 cm) and the catheter was connected to an injection syringe. Human citrated blood was prepared by mixing blood and a solution of 3.8% sodium citrate in a 9:1 ratio. The citrated blood (0.5 ml) was combined with $^{125}$I-fibrinogen (25 $\mu$Ci in 50 $\mu$l of physiological saline solution), 50 $\mu$l of 0.25 M CaCl$_2$ and thrombin (5 U/10 $\mu$l of solution). 16 $\mu$l of the resulting solution was injected into the catheter and the catheter was allowed to stand at room temperature for 60 minutes. The silk thread was then removed from the catheter and washed with a physiological saline solution. The radioactivity bound to the thread (the initial fibrin thrombus value) was determined. The thread was then introduced into a carotid arteriovein (A-V) shunt on a male Sprague-Dawley rat weighing between 200 and 300 grams. One ml samples of the protein in a saline solution containing 50 units heparin per ml were injected into the femoral vein of the animal. After two hours, the silk thread was removed from the shunt and the radioactivity (residual fibrin thrombus value) was determined. The residual thrombus ratio was determined according to the equation:

$$\text{Residual thrombus ratio} = \frac{\text{Residual fibrin thrombus value}}{\text{Initial fibrin thrombus value}} \times 100$$

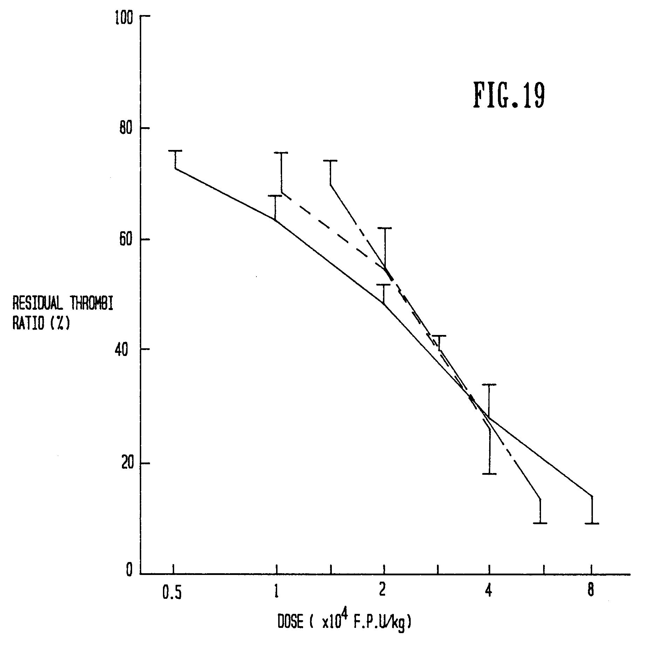

Figure 19 is a graph of the results obtained using various doses of native t-PA, #9100 and #9200. The data indicate that the mutant proteins are comparable to native t-PA in the ability to lyse clots in vivo.

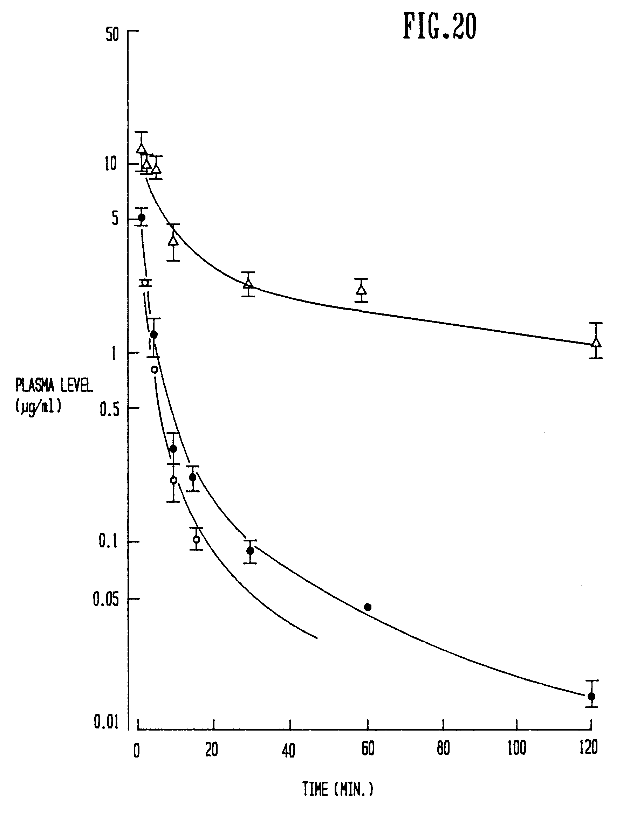

To assay plasma half-life, proteins were solubilized in a saline solution. The solution was injected into the femoral veins of male Sprague-Dawley rats (230 g to 270 g body weight) at a dose of 0.4 mg/kg. Blood samples (0.5 ml) were removed from the jugular veins, adjusted to 3.8% citric acid, and centrifuged. Levels of t-PA in the plasma were determined using a sandwich-type enzyme immunoassay. Figure 20 shows a plot of plasma level vs. time after injection.

Changes in plasma levels of the proteins were analyzed by a two-compartment model (Shargel, L. and Yu, A.B.C., eds., Applied Biopharmaceutics and Pharmacokinetics, Appleton-Century-Crofts, New York, 1980, pp. 38-48). Half-lives were determined for the alpha and beta phases of clearance. The back extrapolated intercept of the beta phase with the ordinate (B) and the area under the curve (AUC) were also determined. The values obtained are presented in Table 9.

## TABLE 9

| Protein | T1/2(α) | T1/2(β) | B | AUC |
|---|---|---|---|---|
| Native t-PA | 1.60 | 31.74 | 0.186 | 33.11 |
| 9200 | 2.22 | 153.22 | 1.780 | 462.95 |

Native t-PA and mutant proteins ZpL7-A, ZpL7-B and ZpL7-F (corresponding to Figure 8A, B and F) were tested for plasma half-life in rats. For each protein tested, two female Balb/c mice weighing 20-21 grams were anesthetized with ether and injected via the tail vein with 10 ug of affinity purified protein in a total volume of 100 ul of PBS. Syringes were weighed before and after injection. At appropriate time intervals, plasma samples (25 ul) were collected from the retroorbital plexus using heparinized micropipettes. The samples were centrifuged to remove red blood cells and diluted for assay. Plasma t-PA levels were measured by ELISA using affinity purified rabbit polyclonal antibody to native human t-PA.

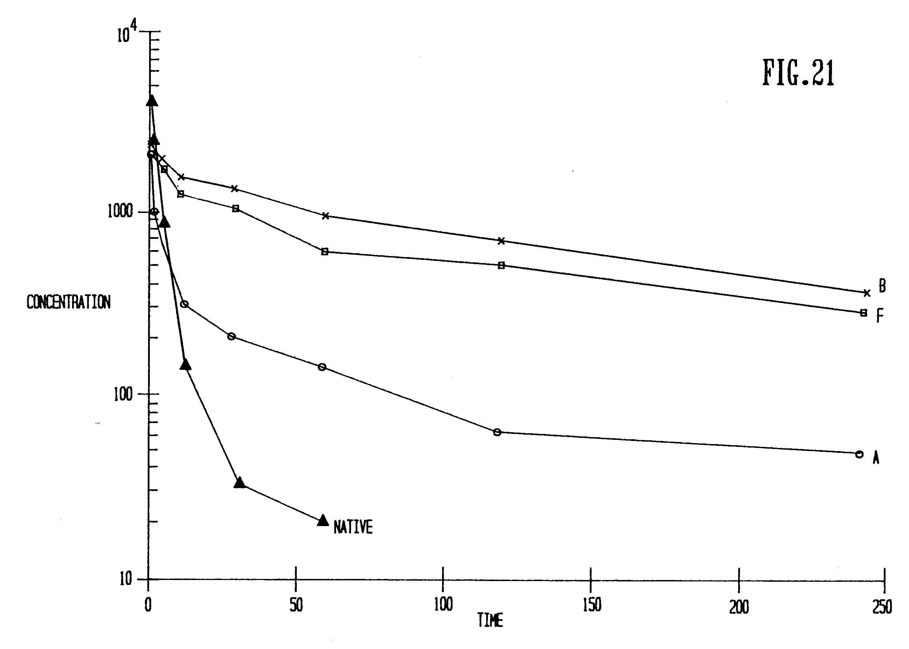

Results of the experiments are shown in Figure 21. All three mutant proteins were found to be cleared at a significantly slower rate than native t-PA. These results suggest that the amino acid changes in the growth factor domain of analog A, and the factor IX and factor VII growth factor substitutions of analogs B and F interfere with the clearance of these molecules by the normal pathway(s).

In similar experiments, the mutant protein ZpL5A (See Example 8) had a half-life of 7.7 minutes. The half-life of native t-PA in these experiments was 1.5 minutes.

Mutant proteins 1601 and 1606 were tested for plasma half-life in rabbits. Results are shown in Table 10.

## TABLE 10

| Protein | T-1/2(minutes |
|---|---|
| Native t-PA (one chain) | 2.3 ± 0.1 |
| Native t-PA (two chain) | 2.0 ± 0.1 |
| 1601 | 3.2 - 3.5 |
| 1606 | 3.2 - 3.0 |

From the foregoing it will be appreciated that, although specific embodiments of the invention have been described herein for purposes of illustration, various modifications may be made without deviating from the spirit and scope of the invention. Accordingly, the invention is not limited except as by the appended claims.

The features disclosed in the foregoing description, in the claims and/or in the accompanying drawings may, both separately and in any combination thereof, be material for realising the invention in diverse forms thereof.

## Claims

1. A t-PA analog containing the growth factor domain of native t-PA, said domain having cysteine residue no. 84 of native t-PA replaced with serine.

EP 0 293 936 B1

2. A t-PA analog containing the growth factor domain of native t-PA, said domain having cysteine residue replaced with serine, wherein the analog further contains a substitution of at least one amino acid within thirteen amino acid residues of the cleavage site, said substitution resulting in an increased resistance to cleavage by plasmin.

3. A t-PA analog containing the growth factor domain of native t-PA, said domain having cysteine residue replaced with serine, wherein the analog contains two kringle structures, at least one of which lacks carbohydrate.

4. A t-PA analog containing the growth factor domain of native t-PA, said domain having cysteine residue replaced with serine, wherein the analog contains a kringle structure derived from plasminogen.

5. A t-PA analog according to Claim 4, wherein the plasminogen kringle structure is selected from the K1, K4 and K5 kringle domains of plasminogen.

6. A t-PA analog according to Claim 5, wherein the analog further contains the K2 kringle structure of native t-PA positioned downstream of the plasminogen kringle structure.

7. A DNA sequence that encodes the t-PA analog containing the growth factor domain of native t-PA, said domain having cysteine residue replaced with serine.

8. A host cell transfected or transformed with an expression vector comprising a DNA sequence according to Claim 7 operably linked to a promoter.

9. A t-PA analog according to any one of Claims 1 to 6, for use as an active therapeutic substance.

10. A t-PA analog according to any one of Claims 1 to 6, for use as a thrombolytic agent.

11. An expression vector containing a DNA sequence as defined in Claim 7.

12. An expression vector according to Claim 11, wherein the vector is Zem 99-9200 (FERM P-9274).

13. A process for manufacturing a t-PA analog which comprises the step of (1) culturing a host cell transformed with an expression vector containing DNA encoding a t-PA analog as defined in Claim 1 and (2) collecting the resultant t-PA analog.

14. The use of a t-PA analog according to Claim 1 for the manufacture of a medicament for the treatment of thrombosis.

15. A t-PA analog having extended half-life, which comprises the t-PA analog of native t-PA in which the cysteine residue at position no. 84 in native t-PA is replaced with serine.

**Patentansprüche**

1. t-PA-Analoges, das den Wachstumsfaktorbereich nativen t-PA's enthält, wobei der Bereich den Cystein-Rest Nr. 84 des nativen t-PA's durch Serin ersetzt hat.

2. t-PA-Analoges, das den Wachstumsfaktorbereich nativen t-PA's enthält, wobei der Bereich einen Cystein-Rest des nativen t-PA's durch Serin ersetzt hat, wobei das Analoge weiterhin eine Substitution wenigstens einer Aminosäure von dreizehn Aminosäureresten der Schneidstelle enthält, wobei die Substitution zu einer erhöhten Resistenz gegen Schneiden durch Plasmin führt.

3. t-PA-Analoges, das den Wachstumsfaktorbereich nativen t-PA's enthält, wobei der Bereich einen Cystein-Rest des nativen t-PA's durch Serin ersetzt hat, wobei das Analoge zwei Kringle-Strukturen enthält, von denen wenigstens ein Kohlenwasserstoff fehlt.

4. t-PA-Analoges, das den Wachstumsfaktorbereich nativen t-PA's enthält, wobei der Bereich einen Cystein-Rest durch Serin ersetzt hat, wobei das Analoge eine Kringle-Struktur enthält, die von

27

EP 0 293 936 B1

Plasminogen abgeleitet ist.

5. t-PA-Analoges nach Anspruch 4, bei dem die Plasminogen-Kringle-Struktur aus den K1-, K4- und K5-Kringle-Bereichen des Plasminogens ausgewählt ist.

6. t-PA-Analoges nach Anspruch 5, bei dem das Analoge weiterhin die K2-Kringle-Struktur nativen t-PA's enthält, stromabwärts der Plasminogen-Kringle-Struktur gelegen.

7. DNA-Sequenz, die das t-PA-Analoge codiert, das den Wachstumsfaktorbereich nativen t-PA's enthält, wobei der Bereich einen Cystein-Rest durch Serin ersetzt hat.

8. Wirtszelle, mit einem Expressionsvektor transfiziert oder transformiert, welcher eine DNA-Sequenz nach Anspruch 7 enthält, operativ mit einem Promoter verbunden.

9. t-PA-Analoges nach einem der Ansprüche 1 bis 6, zur Verwendung als eine aktive therapeutische Substanz.

10. t-PA-Analoges nach einem der Ansprüche 1 bis 6, zur Verwendung als ein thrombolytisches Mittel.

11. Expressionsvektor, der eine DNA-Sequenz wie in Anspruch 7 definiert enthält.

12. Expressionsvektor nach Anspruch 11, bei dem der Vektor Zem 99-9200 (FERM P-9274) ist.

13. Verfahren zum Herstellen eines t-PA-Analogen, welches den Schritt (1) des Züchtens einer Wirtszelle, die mit einem Expressionsvektor transformiert ist, welcher DNA enthält, die ein t-PA-Analoges wie in Anspruch 1 definiert codiert und (2) des Sammelns des sich ergebenden t-PA-Analogen aufweist.

14. Verwendung eines t-PA-Analogen nach Anspruch 1 für die Herstellung eines Medikamentes zur Behandlung von Thrombose.

15. t-PA-Analoges mit verlängerter Halbwertszeit, das das t-PA-Analoge nativen t-PA's enthält, in dem der Cystein-Rest an der Position Nr. 84 in nativem t-PA durch Serin ersetzt ist.

**Revendications**

1. Analogue de t-PA contenant le domaine de facteur de croissance du t-PA naturel, ledit domaine possédant le résidu cystéine n° 84 du t-PA naturel remplacé par la sérine.

2. Analogue de t-PA contenant le domaine de facteur de croissance du t-PA naturel, ledit domaine possédant un résidu cystéine remplacé par la sérine, l'analogue contenant en outre une substitution d'au moins un aminoacide dans treize résidus d'aminoacides du site de clivage, ladite substitution ayant pour résultat une résistance accrue au clivage par la plasmine.

3. Analogue de t-PA contenant le domaine de facteur de croissance du t-PA naturel, ledit domaine possédant un résidu cystéine remplacé par la sérine, ledit analogue contenant deux structures de kringle, dont au moins l'une est dépourvue de glucide.

4. Analogue de t-PA contenant le domaine de facteur de croissance du t-PA naturel, ledit domaine possédant un résidu cystéine remplacé par la sérine, ledit analogue contenant une structure de kringle dérivée du plasminogène.

5. Analogue de t-PA suivant la revendication 4, dans lequel la structure de kringle dérivée du plasminogène est choisie entre les domaines de kringle K1, K4 et K5 du plasminogène.

6. Analogue de t-PA suivant la revendication 5, qui contient en outre la structure de kringle K2 du t-PA naturel positionnée en aval de la structure de kringle du plasminogène.

28

7. Séquence d'ADN qui code pour l'analogue de t-PA contenant le domaine de facteur de croissance du t-PA naturel, ledit domaine possédant un résidu cystéine remplacé par la sérine.

8. Cellule hôte transfectée ou transformée avec un vecteur d'expression comprenant une séquence d'ADN suivant la revendication 7 liée de manière fonctionnelle à un promoteur.

9. Analogue de t-PA suivant l'une quelconque des revendications 1 à 6, destiné à être utilisé comme substance thérapeutique active.

10. Analogue de t-PA suivant l'une quelconque des revendications 1 à 6, destiné à être utilisé comme agent thrombolytique.

11. Vecteur d'expression contenant une séquence d'ADN répondant à la définition suivant la revendication 7.

12. Vecteur d'expression suivant la revendication 11, dans lequel le vecteur est le vecteur Zem 99-9200 (FERM P-9274).

13. Procédé de production d'un anologue de t-PA, qui comprend les étapes consistant (1) à cultiver une cellule hôte transformée avec un vecteur d'expression contenant un ADN codant pour un analogue de t-PA répondant à la définition suivant la revendication 1 et (2) à recueillir l'analogue de t-PA résultant.

14. Utilisation d'un analogue de t-PA suivant la revendication 1 pour la production d'un médicament destiné au traitement d'une thrombose.

15. Analogue de t-PA possédant une demi-vie prolongée, qui comprend l'analogue du t-PA naturel dans lequel le résidu cystéine en position N° 84 dans le t-PA naturel est remplacé par la sérine.

# FIG. 1

```
          10                              30                         45
AAGCTTGGAT CCACC ATG GAT GCA ATG AAG AGA GGG CTC TGC TGT GTG CTG CTG CTG
                 MET Asp Ala MET Lys Arg Gly Leu Cys Cys Val Leu Leu Leu
                 -35                     -30

  60                    75                        90                    105 ·
TGT GGC GCC GTC TTC GTT TCG CCC AGC CAG GAA ATC CAT GCC CGA TTC AGA AGA
Cys Gly Ala Val Phe Val Ser Pro Ser Gln Glu Ile His Ala Arg Phe Arg Arg
    -20               120                     135                   -10
GGA GCC AGA TCT TAC CAA GTC ATC TGC AGA GAT GAA AAA ACG CAG ATG ATA TAC
Gly Ala Arg Ser Tyr Gln Val Ile Cys Arg Asp Glu Lys Thr Gln MET Ile Tyr
            1                                         10

                 180                     195                   210
CAG CAA CAT CAG TCA TGG CTG CGC CCT GTG CTC AGA AGC AAC CGC CTG GAA TAT
Gln Gln His Gln Ser Trp Leu Arg Pro Val Leu Arg Ser Asn Arg Val Glu Tyr
                 20                                    30

    225                   240                     255                   270
TGC TGG TGC AAC AGT GGC AGG GCA CAG TGC CAC TCA GTC CCT GTC AAA AGT TGC
Cys Trp Cys Asm Ser Gly Arg Ala Gln Cys His Ser Val Pro Val Lys Ser Cys
                      40                                          50

                 285                     300                   315
AGC CAG CCA AGG TGT TTC AAC GGG GGC ACC TGC CAG CAG GCC CTG TAC TTC TCA
Ser Gln Pro Arg Cys Phe Asn Gly Gly Thr Cys Gln Gln Ala Leu Tyr Phe Ser
                              60

330                   345                     360                   375
GAT TTC GTG TGC CAG TGC CCC GAA GGA TTT GCT GGG AAG TGC TGT GAA ATA GAT
Asp Phe Val Cys Gln Cys Pro Glu Gly Phe Ala Gly Lys Cys Cys Glu Ile Asp
 70                                           80

            390                     405                   420                   435
ACC AGG GCC AGC TGC TAC GAG GAC CAG GGC ATC AGC TAC AGG GGC ACG TGG AGC
Thr Arg Ala Thr Cys Tyr Glu Asp Gln Gly Ile Ser Tyr Arg Gly Thr Trp Ser
        90                                          100

            450                     465                   480
ACA GCG GAG AGT GGC GCC GAG TGC ACC AAC TGG AAC AGC AGC GCG TTG GCC CAG
Thr Ala Glu Ser Gly Ala Glu Cys Thr Asm Trp Asm Ser Ser Ala Leu Ala Gln
                 110                                   120

    495                   510                     525                   540
AAG CCC TAC AGC GGG CGG AGG CCA CAC GCC ATC AGG CTG GGC CTG CGG AAC CAC
Lys Pro Tyr Ser Gly Arg Arg Pro Asp Ala Ile Arg Leu Gly Leu Gly Asn His
                              130                                   140

            555                     570                   585
AAC TAC TGC AGA AAC CCA GAT CGA GAC TCA AAG CCC TGG TGC TAC GTC TTT AAG
Asm Tyr Cys Arg Asm Pro Asp Arg Asp Ser Lys Pro Trp Cys Tyr Val Phe Lys
                 150

600                   615                     630                   645
GCG GGG AAG TAC AGC TCA GAG TTC TGC AGC ACC CCT GCC TGC TCT CAG GGA AAC
Ala Gly Lys Tyr Ser Ser Glu Phe Cys Ser Thr Pro Ala Cys Ser Glu Gly Asn
160                                           170

            660                     675                   690                   705
AGT GAC TGC TAC TTT GGG AAT GGG TCA GCC TAC CGT GGC ACG CAC AGC CTC ACC
Ser Asp Cys Tyr Phe Gly Asn Gly Ser Ala Tyr Arg Gly Thr His Ser Leu Thr
            180                                   190
```

# FIG. 1 CONT.

```
        720                    735                    750
GAG TCG CGT GCC TCC TGC CTC CCG TGG AAT TCC ATG ATC CTG ATA GCC AAG GTT
Glu Ser Gly Ala Ser Cys Leu Pro Trp Asn Ser MET Ile Leu Ile Gly Lys Val
                200                                   210
    765                    780                    795                    810
TAC ACA GCA CAG AAC CCC AGT GCC CAG GCA CTG GGC CTG GGC AAA CAT AAT TAC
Tyr Thr Ala Gln Asn Pro Ser Ala Gln Ala Leu Gly Leu Gly Lys His Asn Tyr
                        220                                   230
            825                    840                    855
TGC CGG AAT CCT CAT GGG GAT GCC AAG CCC TGG TGC CAC GTG CTG AAG AAC CGC
Cys Arg Asn Pro Asp Gly Asp Ala Lys Pro Trp Cys His Val Leu Lys Asn Arg
                                240
870                    885                    900                    915
AGG CTG ACG TGG GAG TAC TGT GAT GTC CCC TCC TGC TCC ACC TGC GGC CTG AGA
Arg Leu Thr Trp Glu Tyr Cys Asp Val Pro Ser Cys Ser Thr Cys Gly Leu Arg
250                                          260
            930                    945                    960                    975
CAG TAC AGC CAG CCT CAG TTT CGC ATC AAA GGA GGG CTC TTC GCC GAC ATC GCC
Gln Tyr Ser Gln Pro Gln Phe Arg Ile Lys Gly Gly Leu Phe Ala Asp Ile Ala
                270                                   280
            990                    1005                   1020
TCC CAC CCC TGG CAG GCT GCC ATC TTT GCC AAG CAC AGG AGG TCG CCC GGA GAG
Ser His Pro Trp Gln Ala Ala Ile Phe Ala Lys His Arg Arg Ser Pro Gly Glu
                290                                   300
    1035                   1050                   1065                   1080
CGG TTC CTG TGC GGC GGC ATA CTC ATC AGC TCC TGC TGG ATT CTC TCT GCC CCC
Arg Phe Leu Cys Gly Gly Ile Leu Ile Ser Ser Cys Trp Ile Leu Ser Ala Ala
                        310                                   320
        1095                   1110                   1125
CAC TGC TTC CAG GAG AGG TTT CCG CCC CAC CAC CTG ACG GTG ATC TTG GGC AGA
His Cys Phe Gln Glu Arg Phe Pro Pro His His Leu Thr Val Ile Leu Gly Arg
                        330
1140                   1155                   1170                   1185
ACA TAC CGG GTG GTC CCT GGC GAG GAG GAG CAG AAA TTT GAA GTC GAA AAA TAC
Thr Tyr Arg Val Val Pro Gly Glu Glu Glu Gln Lys Phe Glu Val Glu Lys Tyr
340                                          350
    1200                   1215                   1230                   1245
ATT GTC CAT AAG GAA TTC GAT GAT GAC ACT TAC GAC AAT GAC ATT GCG CTG CTG
Ile Val His Lys Glu Phe Asp Asp Asp Thr Tyr Asp Asn Asp Ile Ala Leu Leu
                360                                   370
        1260                   1275                   1290
CAG CTG AAA TCG GAT TCG TCC CGC TGT GCC CAG GAG AGC AGC GTG GTC CGC ACT
Gln Leu Lys Ser Asp Ser Ser Arg Cys Ala Gln Glu Ser Ser Val Val Arg Thr
                380                                   390
    1305                   1320                   1335                   1350
GTG TGC CTT CCC CCG GCG GAC CTG CAG CTG CCG GAC TGG ACG GAG TGT GAG CTC
Val Cys Leu Pro Pro Ala Asp Leu Gln Leu Pro Asp Trp Thr Glu Cys Glu Leu
                        400                                   410
        1365                   1380                   1395
TCC GGC TAC GGC AAG CAT GAG GCC TTG TCT CCT TTC TAT TCG GAG CGG CTC AAG
Ser Gly Tyr Gly Lys His Glu Ala Leu Ser Pro Phe Tyr Ser Glu Arg Leu Lys
                        420
```

31

```
1410                    1425                    1440                    1425
GAG GCT CAT GTC AGA CTG TAC CCA TCC AGC CCC TGC ACA TCA CAA CAT TTA CTT
Glu Ala His Val Arg Leu Tyr Pro Ser Ser Arg Cys Thr Ser Gln His Leu Leu
430                                     440
            1470                    1485                    1500                    1515
AAC AGA ACA GTC ACC GAC AAC ATG CTG TGT GCT GGA GAC ACT CGG AGC GGC GGG
Asn Arg Thr Val Thr Asp Asn MET Leu Cys Ala Gly Asp Thr Arg Ser Gly Gly
            450                                     460
                    1530                    1545                    1560
CCC CAG GCA AAC TTG CAC GAC GCC TGC CAG GCC CAT TCG GGA GGC CCC CTG GTG
Pro Gln Ala Asn Leu His Asp Ala Cys Gln Gly Asp Ser Gly Gly Pro Leu Val
                    470                                     480
        1575                    1590                    1605                    1620
TGT CTG AAC GAT GGC CGC ATG ACT TTG 3TG GGC ATC ATC AGC TGG GGC CTG GGC
Cys Leu Asn Asp Gly Arg MET Thr Leu Val Gly Ile Ile Ser Trp Gly Leu Gly
                        490                                     500 ·
            1635                    1650                    1665
TGT GGA CAG AAG GAT GTC CCG GGT GTG TAC ACA AAG GTT ACC AAC TAC CTA GAC
Cys Gly Gln Lys Asp Val Pro Gly Val Tyr Thr Lys Val Thr Asn Tyr Leu Asp
                                510
1680                    1695                        1714 ¹       1724        1734
TGG ATT CGT GAC AAC ATG CGA CCG TGA CCAGGAACAC CCGACTCCTC AAAAGCAAAT GAGA
Trp Ile Arg Asp Asn MET Arg Pro
520                         527
```

# FIG. 1 CONT.

FIG.2

FIG.3

FIG.4

```
-35  .                -30                                        -20
Met Asp Ala Met Lys Arg Gly Leu Cys Cys Val Leu Leu Leu Cys Gly Ala Val Phe Val
ATG GAT GCA ATG AAG AGA GGG CTC TGC TGT GTG CTG CTG CTG TGT GGC GCC GTC TTC GTT


                      -10                                    -1 +1
Ser Pro Ser Gln Glu Ile His Ala Arg Phe Arg Arg Gly Ala Arg Ser Tyr Gln Val Ile
TCG CCC AGC CAG GAA ATC CAT GCC CGA TTC AGA AGA GGA GCC AGA TCT TAC CAA GTG ATC


                      10                                       20
Cys Arg Asp Glu Lys Thr Gln Met Ile Tyr Gln Gln His Gln Ser Trp Leu Arg Pro Val
TGC AGA GAT GAA AAA ACG CAG ATG ATA TAC CAG CAA CAT CAG TCA TGG CTG CGC CCT GTG


                      30                                       40
Leu Arg Ser Asn Arg Val Glu Tyr Cys Trp Cys Asn Ser Gly Arg Ala Gln Cys His Ser
CTC AGA AGC AAC CGG GTG GAA TAT TGC TGG TGC AAC AGT GGC AGG GCA CAG TGC CAC TCA


                      50                                       60
Val Pro Val Lys Ser Cys Ser Glu Pro Arg Cys Phe Asn Gly Gly Thr Cys Gln Gln Ala
GTG CCT GTC AAA AGT TGC AGC GAG CCA AGG TGT TTC AAC GGG GGC ACC TGC CAG CAG GCC


                      70                                       80
Leu Tyr Phe Ser Asp Phe Val Cys Gln Cys Pro Glu Gly Phe Ala Gly Lys Ser Cys Glu
CTG TAC TTC TCA GAT TTC GTG TGC CAG TGC CCC GAA GGA TTT GCT GGG AAG AGC TGT GAA


                      90                                       100
Ile Asp Thr Arg Ala Thr Cys Tyr Glu Asp Gln Gly Ile Ser Tyr Arg Gly Thr Trp Ser
ATC GAT ACC AGG GCC ACG TGC TAC GAG GAC CAG GGC ATC AGC TAC AGG GGC ACG TGG AGC


                      110                                      120
Thr Ala Glu Ser Gly Ala Glu Cys Thr Asn Trp Asn Ser Ser Ala Leu Ala Gln Lys Pro
ACA GCG GAG AGT GGC GCC GAG TGC ACC AAC TGG AAC AGC AGC GCG TTG GCC CAG AAG CCC


                      130                                      140
Tyr Ser Gly Arg Arg Pro Asp Ala Ile Arg Leu Gly Leu Gly Asn His Asn Tyr Cys Arg
TAC AGC GGG CGG AGG CCA GAC GCC ATC AGC CTG GGC CTG GGG AAC CAC AAC TAC TGC AGA


                      150                                      160
Asn Pro Asp Arg Asp Ser Lys Pro Trp Cys Tyr Val Phe Lys Ala Gly Lys Tyr Ser Ser
AAC CCA GAT CGA GAC TCA AAG CCC TGG TGC TAC GTC TTT AAG GCG GGG AAG TAC AGC TCA


                      170                                      180
Glu Phe Cys Ser Thr Pro Ala Cys Ser Glu Gly Asn Ser Asp Cys Tyr Phe Gly Asn Gly
GAG TTC TGC AGC ACC CCT GCC TGC TCT GAG GGA AAC AGT GAC TGC TAC TTT GGG AAT GGG


                      190                                      200
Ser Ala Tyr Arg Gly Thr His Ser Leu Thr Glu Ser Gly Ala Ser Cys Leu Pro Trp Asn
TCA GCC TAC CGT GGC ACG CAC AGC CTC ACC GAG TCG GGT GCC TCC TGC CTC CCG TGG AAT


                      210                                      220
Ser Met Ile Leu Ile Gly Lys Val Tyr Thr Ala Gln Asn Pro Ser Ala Gln Ala Leu Gly
TCC ATG ATC CTG ATA GGC AAG GTT TAC ACA GCA CAG AAC CCC AGT GCC CAG GCA CTG GGC
```

FIG. 5

36

```
                    230                                    240
Leu Gly Lys His Asn Tyr Cys Arg Asn Pro Asp Gly Asp Ala Lys Pro Trp Cys His Val
CTG GGC AAA CAT AAT TAC TGC CGG AAT CCT GAT GGG GAT GCC AAG CCC TGG TGC CAC GTG


                    250                                    260
Leu Lys Asn Arg Arg Leu Thr Trp Glu Tyr Cys Asp Val Pro Ser Cys Ser Thr Cys Gly
CTG AAG AAC CGC AGG CTG ACG TGG GAG TAC TGT GAT GTG CCC TCC TGC TCC ACC TGC GGC


                    270                                    280
Leu Arg Gln Tyr Ser Gln Pro Gln Phe Arg Ile Lys Gly Gly Leu Phe Ala Asp Ile Ala
CTG AGA CAG TAC AGC CAG CCT CAG TTT CGC ATC AAA GGA GGG CTC TTC GCC GAC ATC GCC


                    290                                    300
Ser His Pro Trp Gln Ala Ala Ile Phe Ala Lys His Arg Arg Ser Pro Gly Glu Arg Phe
TCC CAC CCC TGG CAG GCT GCC ATC TTT GCC AAG CAC AGG AGG TCG CCC GGA GAG CGG TTC


                    310                                    320
Leu Cys Gly Gly Ile Leu Ile Ser Ser Cys Trp Ile Leu Ser Ala Ala His Cys Phe Gln
CTG TGC GGG GGC ATA CTC ATC AGC TCC TGC TGG ATT CTC TCT GCC GCC CAC TGC TTC CAG


                    330                                    340
Glu Arg Phe Pro Pro His His Leu Thr Val Ile Leu Gly Arg Thr Tyr Arg Val Val Pro
GAG AGG TTT CCG CCC CAC CAC CTG ACG GTG ATC TTG GGC AGA ACA TAC CGG GTG GTC CCT


                    350                                    360
Gly Glu Glu Glu Gln Lys Phe Glu Val Glu Lys Tyr Ile Val His Lys Glu Phe Asp Asp
GGC GAG GAG GAG CAG AAA TTT GAA GTC GAA AAA TAC ATT GTC CAT AAG GAA TTC GAT GAT


                    370                                    380
Asp Thr Tyr Asp Asn Asp Ile Ala Leu Leu Gln Leu Lys Ser Asp Ser Ser Arg Cys Ala
GAC ACT TAC GAC AAT GAC ATT GCG CTG CTG CAG CTG AAA TCG GAT TCG TCC CGC TGT GCC


                    390                                    400
Gln Glu Ser Ser Val Val Arg Thr Val Cys Leu Pro Pro Ala Asp Leu Gln Leu Pro Asp
CAG GAG AGC AGC GTG GTC CGC ACT GTG TGC CTT CCC CCG GCG GAC CTG CAG CTG CCG GAC


                    410                                    420
Trp Thr Glu Cys Glu Leu Ser Gly Tyr Gly Lys His Glu Ala Leu Ser Pro Phe Tyr Ser
TGG ACG GAG TGT GAG CTC TCC GGC TAC GGC AAG CAT GAG GCC TTG TCT CCT TTC TAT TCG


                    430                                    440
Glu Arg Leu Lys Glu Ala His Val Arg Leu Tyr Pro Ser Ser Arg Cys Thr Ser Gln His
GAG CGG CTG AAG GAG GCT CAT GTC AGA CTG TAC CCA TCC AGC CGC TGC ACA TCA CAA CAT


                    450                                    460
Leu Leu Asn Arg Thr Val Thr Asp Asn Met Leu Cys Ala Gly Asp Thr Arg Ser Gly Gly
TTA CTT AAC AGA ACA GTC ACC GAC AAC ATG CTG TGT GCT GGA GAC ACT CGG AGC CGC GGG


                    470                                    480
Pro Gln Ala Asn Leu His Asp Ala Cys Gln Gly Asp Ser Gly Gly Pro Leu Val Cys Leu
CCC CAG GCA AAC TTG CAC GAC GCC TGC CAG GGC GAT TCG GGA GGC CCC CTG GTG TGT CTG
```

# FIG. 5 CONT.

```
                    490                                   500
Asn Asp Gly Arg Met Thr Leu Val Gly Ile Ile Ser Trp Gly Leu Gly Cys Gly Gln Lys
AAC GAT GGC CGC ATG ACT TTG GTG GGC ATC ATC AGC TGG GGC CTG GGC TGT GGA CAG AAG

                    510                                   520
Asp Val Pro Gly Val Tyr Thr Lys Val Thr Asn Tyr Leu Asp Trp Ile Arg Asp Asn Met
GAT GTC CCG GGT GTG TAC ACA AAG GTT ACC AAC TAC CTA GAC TGG ATT CGT GAC AAC ATG

    527
Arg Pro ---
CGA CCG TGA
```

# FIG.5 CONT.

```
-35                      -30                                    -20
Met Asp Ala Met Lys Arg Gly Leu Cys Cys Val Leu Leu Leu Cys Gly Ala Val Phe Val
ATG GAT GCA ATG AAG AGA GGG CTC TGC TGT GTG CTG CTG CTG TGT GGC GCC GTC TTC GTT


                         -10                                    -1  +1
Ser Pro Ser Gln Glu Ile His Ala Arg Phe Arg Arg Gly Ala Arg Ser Tyr Gln Val Ile
TCG CCC AGC CAG GAA ATC CAT GCC CGA TTC AGA AGA GGA GCC AGA TCT TAC CAA GTG ATC


                         10                                     20
Cys Arg Asp Glu Lys Thr Gln Met Ile Tyr Gln Gln His Gln Ser Trp Leu Arg Pro Val
TGC AGA GAT GAA AAA ACG CAG ATG ATA TAC CAG CAA CAT CAG TCA TGG CTG CGC CCT GTG


                         30                                     40
Leu Arg Ser Asn Arg Val Glu Tyr Cys Trp Cys Asn Ser Gly Arg Ala Gln Cys His Ser
CTC AGA AGC AAC CGG GTG GAA TAT TGC TGG TGC AAC AGT GGC AGG GCA CAG TGC CAC TCA


                         50                                     60
Val Pro Val Lys Ser Cys Ser Glu Pro Arg Cys Phe Asn Gly Gly Thr Cys Gln Gln Ala
GTG CCT GTC AAA AGT TGC AGC GAG CCA AGG TGT TTC AAC GGG GGC ACC TGC CAG CAG GCC


                         70                                     80
Leu Tyr Phe Ser Asp Phe Val Cys Gln Cys Pro Glu Gly Phe Ala Gly Lys Cys Ser Glu
CTG TAC TTC TCA GAT TTC GTG TGC CAG TGC CCC GAA GGA TTT GCT GGG AAG TGC AGT GAA


                         90                                     100
Ile Asp Thr Arg Ala Thr Cys Tyr Glu Asp Gln Gly Ile Ser Tyr Arg Gly Thr Trp Ser
ATC GAT ACC AGG GCC ACG TGC TAC GAG GAC CAG GGC ATC AGC TAC AGG GGC ACG TGG AGC


                         110                                    120
Thr Ala Glu Ser Gly Ala Glu Cys Thr Asn Trp Asn Ser Ser Ala Leu Ala Gln Lys Pro
ACA GCG GAG AGT GGC GCC GAG TGC ACC AAC TGG AAC AGC AGC GCG TTG GCC CAG AAG CCC


                         130                                    140
Tyr Ser Gly Arg Arg Pro Asp Ala Ile Arg Leu Gly Leu Gly Asn His Asn Tyr Cys Arg
TAC AGC GGG CGG AGG CCA GAC GCC ATC AGG CTG GGC CTG GGG AAC CAC AAC TAC TGC AGA


                         150                                    160
Asn Pro Asp Arg Asp Ser Lys Pro Trp Cys Tyr Val Phe Lys Ala Gly Lys Tyr Ser Ser
AAC CCA GAT CGA GAC TCA AAG CCC TGG TGC TAC GTC TTT AAG GCG GGG AAG TAC AGC TCA


                         170                                    180
Glu Phe Cys Ser Thr Pro Ala Cys Ser Glu Gly Asn Ser Asp Cys Tyr Phe Gly Asn Gly
GAG TTC TGC AGC ACC CCT GCC TGC TCT GAG GGA AAC AGT GAC TGC TAC TTT GGG AAT GGG


                         190                                    200
Ser Ala Tyr Arg Gly Thr His Ser Leu Thr Glu Ser Gly Ala Ser Cys Leu Pro Trp Asn
TCA GCC TAC CGT GGC ACG CAC AGC CTC ACC GAG TCG GGT GCC TCC TGC CTC CCG TGG AAT


                         210                                    220
Ser Met Ile Leu Ile Gly Lys Val Tyr Thr Ala Gln Asn Pro Ser Ala Gln Ala Leu Gly
TCC ATG ATC CTG ATA GGC AAG GTT TAC ACA GCA CAG AAC CGC AGT GCC CAG GCA CTG GGC
```

# FIG.6

```
                     230                              240
Leu Gly Lys His Asn Tyr Cys Arg Asn Pro Asp Gly Asp Ala Lys Pro Trp Cys His Val
CTG GGC AAA CAT AAT TAC TGC CGG AAT CCT GAT GGG GAT GCC AAG CCC TGG TGC CAC GTG

            250                              260
Leu Lys Asn Arg Arg Leu Thr Trp Glu Tyr Cys Asp Val Pro Ser Cys Ser Thr Cys Gly
CTG AAG AAC CGC AGG CTG ACG TGG GAG TAC TGT GAT GTG CCC TCC TGC TCC ACC·TGC GGC

            270                              280
Leu Arg Gln Tyr Ser Gln Pro Gln Phe Arg Ile Lys Gly Gly Leu Phe Ala Asp Ile Ala
CTG AGA CAG TAC AGC CAG CCT CAG TTT CGC ATC AAA GGA GGG CTC TTC GCC GAC ATC GCC

            290                              300
Ser His Pro Trp Gln Ala Ala Ile Phe Ala Lys His Arg Arg Ser Pro Gly Glu Arg Phe
TCC CAC CCC TGG CAG GCT GCC ATC TTT GCC AAG CAC AGG AGG TCG CCC GGA GAG CGG TTC

            310                              320
Leu Cys Gly Gly Ile Leu Ile Ser Ser Cys Trp Ile Leu Ser Ala Ala His Cys Phe Gln
CTG TGC GGG GGC ATA CTC ATC AGC TCC TGC TGG ATT CTC TCT GCC GCC CAC TGC TTC CAG

            330                              340
Glu Arg Phe Pro Pro His His Leu Thr Val Ile Leu Gly Arg Thr Tyr Arg Val Val Pro
GAG AGG TTT CCG CCC CAC CAC CTG ACG GTG ATC TTG GGC AGA ACA TAC CGG GTG GTC CCT

            350                              360
Gly Glu Glu Glu Gln Lys Phe Glu Val Glu Lys Tyr Ile Val His Lys Glu Phe Asp Asp
GGC GAG GAG GAG CAG AAA TTT GAA GTC GAA AAA TAC ATT GTC CAT AAG GAA TTC GAT GAT

            370                              380
Asp Thr Tyr Asp Asn Asp Ile Ala Leu Leu Gln Leu Lys Ser Asp Ser Ser Arg Cys Ala
GAC ACT TAC GAC AAT GAC ATT GCG CTG CTG CAG CTG AAA TCG GAT TCG TCC CGC TGT GCC

            390                              400
Gln Glu Ser Ser Val Val Arg Thr Val Cys Leu Pro Pro Ala Asp Leu Gln Leu Pro Asp
CAG GAG AGC AGC GTG GTC CGC ACT GTG TGC CTT CCC CCG GCG GAC CTG CAG CTG CCG GAC

            410                              420
Trp Thr Glu Cys Glu Leu Ser Gly Tyr Gly Lys His Glu Ala Leu Ser Pro Phe Tyr Ser
TGG ACG GAG TGT GAG CTC TCC GGC TAC GGC AAG CAT GAG GCC TTG TCT CCT TTC TAT TCG

            430                              440
Glu Arg Leu Lys Glu Ala His Val Arg Leu Tyr Pro Ser Ser Arg Cys Thr Ser Gln His
GAG CGG CTG AAG GAG GCT CAT GTC AGA CTG TAC CCA TCC AGC CGC TGC ACA TCA CAA CAT

            450                              460
Leu Leu Asn Arg Thr Val Thr Asp Asn Met Leu Cys Ala Gly Asp Thr Arg Ser Gly Gly
TTA CTT AAC AGA ACA GTC ACC GAC AAC ATG CTG TGT GCT GGA GAC ACT CGG AGC GGC GGG

            470                              480
Pro Gln Ala Asn Leu His Asp Ala Cys Gln Gly Asp Ser Gly Gly Pro Leu Val Cys Leu
CCC CAG GCA AAC TTG CAC GAC GCC TGC CAG GGC GAT TCG GGA GGC CCC CTG GTG TGT CTG
```

FIG. 6 CONT.

```
                    490                                500
Asn Asp Gly Arg Met Thr Leu Val Gly Ile Ile Ser Trp Gly Leu Gly Cys Gly Gln Lys
AAC GAT GGC CGC ATG ACT TTG GTG GGC ATC ATC AGC TGG GGC CTG GGC TGT GGA CAG AAG

                    510                                520
Asp Val Pro Gly Val Tyr Thr Lys Val Thr Asn Tyr Leu Asp Trp Ile Arg Asp Asn Met
GAT GTC CCG GGT GTG TAC ACA AAG GTT ACC AAC TAC CTA GAC TGG ATT CGT GAC AAC ATG

     527
Arg Pro ---
CGA CCG TGA
```

# FIG.6 CONT.

FIG.7

# FIG.8

FIG.9

IOA     CRDEKTQMIYQQHQSWLRPVLRSNRVEYCWCNSGRAQC

IOB     CFDNGKSYKIGETWERPYEGFMLSCTCLGNGRGEFRC

IOC     CHDEKTGSSYKIGEQWERPYLSGNRLECTCLGNGSGRWQC

IOD     CFDNGKSYKIGETWERPYEGFMLSCTCLGNGSGRWQC

IOE     CFDNGKSYKIGEQWERPYLSGNRLECTCLGNGRGEFRC

IOF     CFDNGKSYKIGEQWERPYLSGNRLECTCLGNGSGRWQC

IOG     CHDEKTGSSYKIGETWERPYEGFMLSCTCLGNGSGRWQC

IOH     CHDEKTGSSYKIGEQWERPYLSGNRLECTCLGNGRGEFRC

IOI     CHDEKTGSSYKIGETWERPYEGFMLSCTCLGNGRGEFRC

FIG. 10

45

```
Cys Lys Thr Gly Asp Gly Lys Asn Tyr Arg Gly Thr Met Ser
TGC AAG ACC GGT GAT GGT AAA AAC TAC CGA GGT ACC ATG TCC


Lys Thr Lys Asn Gly Ile Thr Cys Gln Lys Trp Ser Ser Thr Ser Pro His Arg Pro Arg
AAG ACC AAA AAC GGT ATT ACA TGT CAG AAA TGG TCA TCT ACT AGT CCA CAC CGG CCG CGG


Phe Ser Pro Ala Thr His Pro Ser Glu Gly Leu Glu Glu Asn Tyr Cys Arg Asn Pro Asp
TTT TCT CCA GCT ACC CAT CCA TCT GAA GGC CTG GAA GAG AAT TAC TGT AGG AAT CCA GAT


Asn Asp Pro Gln Gly Pro Trp Cys Tyr Thr Thr Asp Pro Glu Lys Arg Tyr Asp Tyr Cys
AAC GAT CCT CAG GGT CCC TGG TGT TAC ACC ACA GAC CCC GAG AAG AGG TAC GAC TAC TGC


Asp Ile Leu Glu Cys
GAT ATC CTG GAA TGC
```

FIG. 11

EP 0 293 936 B1

20bp

| 1 | 50 | 121 | 182 | 250 |
|---|----|-----|-----|-----|
| Mlu I | Kpn I | Fok I | Sau 3AI | Eco RV |

#1-3

#8-5

FIG.12

FIG.13

FIG.14

## FIG.15

# FIG.16

```
-35                      -30                                                    -20
Met Asp Ala Met Lys Arg Gly Leu Cys Cys Val Leu Leu Leu Cys Gly Ala Val Phe Val
ATG GAT GCA ATG AAG AGA GGG CTC TGC TGT GTG CTG CTG CTG TGT GGC GCC GTC TTC GTT

                         -10                                           -1  +1
Ser Pro Ser Gln Glu Ile His Ala Arg Phe Arg Arg Gly Ala Arg Ser Tyr Gln Val Ile
TCG CCC AGC CAG GAA ATC CAT GCC CGA TTC AGA AGA GGA GCC AGA TCT TAC CAA GTG ATC

                          10                                      20
Cys Arg Asp Glu Lys Thr Gln Met Ile Tyr Gln Gln His Gln Ser Trp Leu Arg Pro Val
TGC AGA GAT GAA AAA ACG CAG ATG ATA TAC CAG CAA CAT CAG TCA TGG CTG CGC CCT GTG

                          30                                      40
Leu Arg Ser Asn Arg Val Glu Tyr Cys Trp Cys Asn Ser Gly Arg Ala Gln Cys His Ser
CTC AGA AGC AAC CGG GTG GAA TAT TGC TGG TGC AAC AGT GGC ACG GCA CAG TGC CAC TCA

                          50                                      60
Val Pro Val Lys Ser Cys Ser Glu Pro Arg Cys Phe Asn Gly Gly Thr Cys Gln Gln Ala
GTG CCT GTC AAA AGT TGC AGC GAG CCA AGG TGT TTC AAC GGG GGC ACC TGC CAG CAG GCC

                          70                                      80
Leu Tyr Phe Ser Asp Phe Val Cys Gln Cys Pro Glu Gly Phe Ala Gly Lys Cys Cys Glu
CTG TAC TTC TCA GAT TTC GTG TGC CAG TGC CCC GAA GGA TTT GCT GGG AAG TGC TGT GAA

                          90                                      100
Ile Asp Thr Arg Ala Thr Cys Lys Thr Gly Asp Gly Lys Asn Tyr Arg Gly Thr Met Ser
ATA GAT ACG CGT GCC ACG TGC AAG ACC GGT GAT GGT AAA AAC TAC CGA GGT ACC ATG TCC

                          110                                     120
Lys Thr Lys Asn Gly Ile Thr Cys Gln Lys Trp Ser Ser Thr Ser Pro His Arg Pro Arg
AAG ACC AAA AAC GGT ATT ACA TGT CAG AAA TGG TCA TCT ACT AGT CCA CAC CGG CCG CGG

                          130                                     140
Phe Ser Pro Ala Thr His Pro Ser Glu Gly Leu Glu Glu Asn Tyr Cys Arg Asn Pro Asp
TTT TCT CCA GCT ACC CAT CCA TCT GAA GGC CTG GAA GAG AAT TAC TGT AGG AAT CCA GAT

                          150                                     160
Asn Asp Pro Gln Gly Pro Trp Cys Tyr Thr Thr Asp Pro Glu Lys Arg Tyr Asp Tyr Cys
AAC GAT CCT CAG GGT CCC TGG TGT TAC ACC ACA GAC CCC GAG AAG AGG TAC GAC TAC TGC

                          170                                     180
Asp Ile Leu Glu Cys Ser Glu Gly Asn Ser Asp Cys Tyr Phe Gly Asn Gly Ser Ala Tyr
GAT ATC CTG GAA TGC TCT GAG GGA AAC AGT GAC TGC TAC TTT GGG AAT GGG TCA GCC TAC

                          190                                     200
Arg Gly Thr His Ser Leu Thr Glu Ser Gly Ala Ser Cys Leu Pro Trp Asn Ser Met Ile
CGT GGC ACG CAC AGC CTC ACC GAG TCG GGT GCC TCC TGC CTC CCG TGG AAT TCC ATG ATC

                          210                                     220
Leu Ile Gly Lys Val Tyr Thr Ala Gln Asn Pro Ser Ala Gln Ala Leu Gly Leu Gly Lys
CTG ATA GGC AAG GTT TAC ACA GCA CAG AAC CCC AGT GCC CAG GCA CTG GGC CTG GGC AAA
```

## FIG. 17

```
                    230                                240
His Asn Tyr Cys Arg Asn Pro Asp Gly Asp Ala Lys Pro Trp Cys His Val Leu Lys Asn
CAT AAT TAC TGC CGG AAT CCT GAT GGG GAT GCC AAG CCC TGG TGC CAC GTG CTG AAG AAC

                    250                                260
Arg Arg Leu Thr Trp Glu Tyr Cys Asp Val Pro Ser Cys Ser Thr Cys Gly Leu Arg Gln
CGC AGG CTG ACG TGG GAG TAC TGT GAT GTG CCC TCC TGC TCC ACC TGC GGC CTG AGA CAG

                    270                                280
Tyr Ser Gln Pro Gln Phe Arg Ile Lys Gly.Gly Leu Phe Ala Asp Ile Ala Ser His Pro
TAC AGC CAG CCT CAG TTT CGC ATC AAA GGA GGG CTC TTC GCC GAC ATC GCC TCC CAC CCC

                    290                                300
Trp Gln Ala Ala Ile Phe Ala Lys His Arg Arg Ser Pro Gly Glu Arg Phe Leu Cys Gly
TGG CAG GCT GCC ATC TTT GCC AAG CAC AGG AGG TCG CCC GGA GAG CGG TTC CTG TGC GGG

                    310                                320
Gly Ile Leu Ile Ser Ser Cys Trp Ile Leu Ser Ala Ala His Cys Phe Gln Glu Arg Phe
GGC ATA CTC ATC AGC TCC TGC TGG ATT CTC TCT GCC GCC CAC TGC TTC CAG GAG AGG TTT

                    330                                340
Pro Pro His His Leu Thr Val Ile Leu Gly Arg Thr Tyr Arg Val Val Pro Gly Glu Glu
CCG CCC CAC CAC CTG ACG GTG ATC TTG GGC AGA ACA TAC CGG GTG GTC CCT GGC GAG GAG

                    350                                360
Glu Gln Lys Phe Glu Val Glu Lys Tyr Ile Val His Lys Glu Phe Asp Asp Asp Thr Tyr
GAG CAG AAA TTT GAA GTC GAA AAA TAC ATT GTC CAT AAG GAA TTC GAT GAT GAC ACT TAC

                    370                                380
Asp Asn Asp Ile Ala Leu Leu Gln Leu Lys Ser Asp Ser Ser Arg Cys Ala Gln Glu Ser
GAC AAT GAC ATT GCG CTG CTG CAG CTG AAA TCG GAT TCG TCC CGC TGT GCC CAG GAG AGC

                    390                                400
Ser Val Val Arg Thr Val Cys Leu Pro Pro Ala Asp Leu Gln Leu Pro Asp Trp Thr Glu
AGC GTG GTC CGC ACT GTG TGC CTT CCC CCG GCG GAC CTG CAG CTG CCG GAC TGG ACG GAG

                    410                                420
Cys Glu Leu Ser Gly Tyr Gly Lys His Glu Ala Leu Ser Pro Phe Tyr Ser Glu Arg Leu
TGT GAG CTC TCC GGC TAC GGC AAG CAT GAG GCC TTG TCT CCT TTC TAT TCG GAG CGG CTG

                    430                                440
Lys Glu Ala His Val Arg Leu Tyr Pro Ser Ser Arg Cys Thr Ser Gln His Leu Leu Asn
AAG GAG GCT CAT GTC AGA CTG TAC CCA TCC AGC CGC TGC ACA TCA CAA CAT TTA CTT AAC

                    450                                460
Arg Thr Val Thr Asp Asn Met Leu Cys Ala Gly Asp Thr Arg Ser Gly Gly Pro Gln Ala
AGA ACA GTC ACC GAC AAC ATG CTG TGT GCT GGA GAC ACT CGG AGC GGC GGG CCC CAG GCA

                    470                                480
Asn Leu His Asp Ala Cys Gln Gly Asp Ser Gly Gly Pro Leu Val Cys Leu Asn Asp Gly
AAC TTG CAC GAC GCC TGC CAG GGC GAT TCG GGA GGC CCC CTG GTG TGT CTG AAC GAT GCC
```

## FIG.17 CONT.

```
                   490                                    500
   Arg Met Thr Leu Val Gly Ile Ile Ser Trp Gly Leu Gly Cys Gly Gln Lys Asp Val Pro
   CGC ATG ACT TTG GTG GGC ATC ATC AGC TGG GGC CTG GGC TGT GGA CAG AAG GAT GTC CCG

                   510                                    520        524
   Gly Val Tyr Thr Lys Val Thr Asn Tyr Leu Asp Trp Ile Arg Asp Asn Met Arg Pro ---
   GGT GTG TAC ACA AAG GTT ACC AAC TAC CTA GAC TGG ATT CGT GAC AAC ATG CGA CCG TGA
```

# FIG.17 CONT.

```
-35                    -30                                                -20
Met Asp Ala Met Lys Arg Gly Leu Cys Cys Val Leu Leu Leu Cys Gly Ala Val Phe Val
ATG GAT CCA ATG AAG AGA GGG CTC TGC TGT GTG CTG CTG CTG TGT GGC GCC GTC TTC GTT

                      -10                                         -1 +1
Ser Pro Ser Gln Glu Ile His Ala Arg Phe Arg Arg Gly Ala Arg Ser Tyr Gln Val Ile
TCG CCC AGC CAG GAA ATC CAT GCC CGA TTC AGA AGA GGA GCC AGA TCT TAC CAA GTG ATC

                      10                                          20
Cys Arg Asp Glu Lys Thr Gln Met Ile Tyr Gln Gln His Gln Ser Trp Leu Arg Pro Val
TGC AGA GAT GAA AAA ACG CAG ATG ATA TAC CAG CAA CAT CAG TCA TGG CTG CGC CCT GTG

                      30                         .               40
Leu Arg Ser Asn Arg Val Glu Tyr Cys Trp Cys Asn Ser Gly Arg Ala Gln Cys His Ser
CTC AGA AGC AAC CGG GTG GAA TAT TGC TGG TGC AAC AGT GGC AGG GCA CAG TGC CAC TCA

                      50                                          60
Val Pro Val Lys Ser Cys Ser Glu Pro Arg Cys Phe Asn Gly Gly Thr Cys Gln Gln Ala
GTG CCT GTC AAA AGT TGC AGC GAG CCA AGG TGT TTC AAC GGG GGC ACC TGC CAG CAG GCC

                      70                                          80
Leu Tyr Phe Ser Asp Phe Val Cys Gln Cys Pro Glu Gly Phe Ala Gly Lys Cys Cys Glu
CTG TAC TTC TCA GAT TTC GTG TGC CAG TGC CCC GAA GGA TTT GCT GGG AAG TGC TGT GAA

                      90                                          100
Ile Asp Thr Arg Ala Thr Cys Lys Thr Gly Asp Gly Lys Asn Tyr Arg Gly Thr Met Ser
ATA GAT ACG CGT GCC ACG TGC AAG ACC GGT GAT GGT AAA AAC TAC CGA GGT ACC ATG TCC

                      110                                         120
Lys Thr Lys Asn Gly Ile Thr Cys Gln Lys Trp Ser Ser Thr Ser Pro His Arg Pro Arg
AAG ACC AAA AAC GGT ATT ACA TGT CAG AAA TGG TCA TCT ACT AGT CCA CAC CGG CCG CGG

                      130                        .               140
Phe Ser Pro Ala Thr His Pro Ser Glu Gly Leu Glu Glu Asn Tyr Cys Arg Asn Pro Asp
TTT TCT CCA GCT ACC CAT CCA TCT GAA GGC CTG GAA GAG AAT TAC TGT AGG AAT CCA GAT

                      150                                         160
Asn Asp Pro Gln Gly Pro Trp Cys Tyr Thr Thr Asp Pro Glu Lys Arg Tyr Asp Tyr Cys
AAC GAT CCT CAG GGT CCC TGG TGT TAC ACC ACA GAC CCC GAG AAG AGG TAG GAC TAC TGC

                      170                                         180
Asp Ile Leu Glu Cys Ser Glu Gly Asn Ser Asp Cys Tyr Phe Gly Asn Gly Ser Ala Tyr
GAT ATC CTG GAA TGC TCT GAG GGA AAC AGT GAC TGC TAC TTT GGG AAT GGG TCA GCC TAC

                      190                                         200
Arg Gly Thr His Ser Leu Thr Glu Ser Gly Ala Ser Cys Leu Pro Trp Asn Ser Met Ile
CGT GGC ACG CAC AGC CTC ACC GAG TCG GGT GCC TCC TGC CTC CCG TGG AAT TCC ATG ATC

                      210                                         220
Leu Ile Gly Lys Val Tyr Thr Ala Gln Asn Pro Ser Ala Gln Ala Leu Gly Leu Gly Lys
CTG ATA GGC AAG GTT TAG ACA GCA CAG AAC CCC AGT GCC CAG GCA CTG GGC CTC GGC AAA
```

FIG.18

```
                230                                          240
His Asn Tyr Cys Arg Asn Pro Asp Gly Asp Ala Lys Pro Trp Cys His Val Leu Lys Asn
CAT AAT TAC TGC CGG AAT CCT GAT GGG GAT GCC AAG CCC TGG TGC CAC GTG CTG AAG AAC


                250                                          260
Arg Arg Leu Thr Trp Glu Tyr Cys Asp Val Pro Ser Cys Ser Thr Cys Gly Leu Arg Gln
CGC AGG CTG ACG TGG GAG TAC TGT GAT GTG CCC TCC TGC TCC ACC TGC GGC CTG AGA CAG


                270                                          280
Tyr Ser Gln Pro Gln Phe Arg Ile Lys Gly Gly Leu Phe Ala Asp Ile Ala Ser His Pro
TAC AGC CAG CCT CAG TTT CGC ATC AAA GGA GGG CTC TTC GCC GAC ATC GCC TCC CAC CCC


                290                                          300
Trp Gln Ala Ala Ile Phe Ala Lys His Arg Arg Ser Pro Gly Glu Arg Phe Leu Cys Gly
TGG CAG GCT GCC ATC TTT GCC AAG CAC AGG AGG TCG CCC GGA GAG CGG TTC CTG TGC GGG


                310                                          320
Gly Ile Leu Ile Ser Ser Cys Trp Ile Leu Ser Ala Ala His Cys Phe Gln Glu Arg Phe
GGC ATA CTC ATC AGC TCC TGC TGG ATT CTC TCT GCC GCC CAC TGC TTC CAG GAG AGG TTT


                330                                          340
Pro Pro His His Leu Thr Val Ile Leu Gly Arg Thr Tyr Arg Val Val Pro Gly Glu Glu
CCG CCC CAC CAC CTG ACG GTG ATC TTG GGC AGA ACA TAC CGG GTG GTC CCT GGC GAG GAG


                350                                          360
Glu Gln Lys Phe Glu Val Glu Lys Tyr Ile Val His Lys Glu Phe Asp Asp Asp Thr Tyr
GAG CAG AAA TTT GAA GTC GAA AAA TAC ATT GTC CAT AAG GAA TTC GAT GAT GAC ACT TAC


                370                                          380
Asp Asn Asp Ile Ala Leu Leu Gln Leu Lys Ser Asp Ser Ser Arg Cys Ala Gln Glu Ser
GAC AAT GAC ATT GCG CTG CTG CAG CTG AAA TCG GAT TCG TCC CGC TGT GCC CAG GAG AGC


                390                                          400
Ser Val Val Arg Thr Val Cys Leu Pro Pro Ala Asp Leu Gln Leu Pro Asp Trp Thr Glu
AGC GTG GTC CGC ACT GTG TGC CTT CCC CCG GCG GAC CTG CAG CTG CCG GAC TGG ACG GAG


                410                                          420
Cys Glu Leu Ser Gly Tyr Gly Lys His Glu Ala Leu Ser Pro Phe Tyr Ser Glu Arg Leu
TGT GAG CTC TCC GGC TAC GGC AAG CAT GAG GCC TTG TCT CCT TTC TAT TCG GAG CGG CTG


                430                                          440
Lys Glu Ala His Val Arg Leu Tyr Pro Ser Ser Arg Cys Thr Ser Gln His Leu Leu Asn
AAG GAG GCT CAT GTC AGA CTG TAC CCA TCC AGC CGC TGC ACA TCA CAA CAT TTA CTT AAC


                450                                          460
Arg Thr Val Thr Asp Asn Met Leu Cys Ala Gly Asp Thr Arg Ser Gly Gly Pro Gln Ala
AGA ACA GTC ACC GAC AAC ATG CTG TGT GCT GGA GAC ACT CGG AGC GGC GGG CCC CAG GCA


                470                                          480
Asn Leu His Asp Ala Cys Gln Gly Asp Ser Gly Gly Pro Leu Val Cys Leu Asn Asp Gly
AAC TTG CAC GAC GCC TGC CAG GGC GAT TCG GGA GGC CCC CTG GTG TGT CTG AAC GAT GGC
```

FIG. 18 CONT.

```
                      490                                   500
Arg Met Thr Leu Val Gly Ile Ile Ser Trp Gly Leu Gly Cys Gly Gln Lys Asp Val Pro
CGC ATG ACT TTG GTG GGC ATC ATC AGC TGG GGC CTG GGC TGT GGA CAG AAG GAT GTC CCG

                      510                                   520        524
Gly Val Tyr Thr Lys Val Thr Asn Tyr Leu Asp Trp Ile Arg Asp Asn Met Arg Pro ----
GGT GTG TAC ACA AAG GTT ACC AAC TAC CTA GAC TGG ATT CGT GAC AAC ATG CGA CCG TGA
```

# FIG. 18 CONT.

FIG.19

FIG.20

FIG.21

EP 0 293 936 B1

```
TPA:      CS-E--PR---------CFNGGT-------CQQALYFSD----FVCQC-PEG---F---A-GKCC
Urokh:    C--D------------CLNGGT-------CVSNKYFSNI--HW-CNC-P--KK-F---G-GQHC
Urokp:    C---------------GCLNGGK-------CVSYKYFSNI-QR--CSC-P--KK-F---Q-GEHC
Urokm:    C---------------GCQNGGV-------CVSYKYFSRI-RR--CSC-P--RK-F---Q-GEHC
F7-4h:    CA--SSP---------CQNGGS-------CKDQLQS------YICFCLPA----F-----EGRNC
F9-4h:    C--ESNP---------CLNGGS-------CKDDINS------YECWC-PFG---F-----EGKNC
F9-4b:    C--ESNP---------CLNGGM-------CKDDINS------YECWCQ--AG---F-----EGTNC
F10-4h:   C--ETSP---------CQNQGK-------CKDGLGE------YTCTCL-EG---F-----EGKNC
F10-4b:   C--EGHP---------CLNQGH-------CKDGIGD----;-YTCTCA-EG---F-----EGKNC
PS-4Ah:   CS-----PL--P-----CNEDGYMS-----CKDGKAS------FTCTKP-G---W---Q-GEKC
PS-4Ab:   CN-----PL--P-----CNEDGFMT-----CKDGQAT------FTCICK-SG---W---Q-GEKC
PZ-4b:    CA--SQP---------CLNNGS-------CQDSIRG------YACTCAP-G---Y-----EGPNC
EGFh:     C-----PLSH---DGYCLHDGV-------CMYIEALD----KYACNCV-VG---YIG--E-R-C
EGFm:     C-----PSSY---DGYCLNGGV-------CMHIESLDS----YTCNCV-IG---YSG--D-R-C
TGFah:    C-----PDSHTQF---CFHGT-------CRFLVQED---KPA-CVCH-SG---YVGA---R-C
TGFar:    C-----PDSHTQY---CFHGT-------CRFLVQEE---KPA-CVCH-SG---YVGV---R-C
TGFIr:    C-----PDSHTQY---CFHGT-------CRFLVQEE---KPA-CVCH-SG---YVGV---R-C
C9:       C--------HT-----CQNGGTVILMDGKCL--------------CAC-PF-K--F----EGIAC
```

# FIG.22A

EP 0 293 936 B1

```
F7-5:      CVNE------------NGGC-EQ-Y--------CSDHTGT----KRS-CRCH-EG---YSLLADGVSC
F9-5h:     CN-IK-----------NGRC-EQ-F--------CKNSADN----KVV-CSCT-EG---YRLAENQKSC
F9-5b:     CS-IK-----------NGRC-KQ-F--------CKRDTDN----KVV-CSCT-DG---YRLAEDQKSC
F10-5h:    CSLD------------NGDC-DQ-F--------CHEEQNS-----VV-CSCA-RG---YTLADNGKAC
F10-5b:    CSLD------------NGGC-DQ-F--------CREERSE----VR--CSCA-HG---YVLGDDSKSC
PC-5h:     CSLD------------NGGCTH--Y--------CLEEVGW----RR--CSCAP-G---YKLGDDLLQC
PC-5b:     CSAE------------NGGCAH--Y--------CMEEEG-----RRH-CSCAP-G---YRLEDDHQLC
PS-5Ah:    CK-D--PSNI---NGGC-SQI--------CDNTPGS------YHCSCK-NG---FVMLSNKKDC
PS-5Ab:    CK-D--PVNI---NGGC-SQI--------CENTPGS------YHCSCK-NG-  -FVMLSNKKDC
EGFr1m:    CATQ------------NHGCTLG--------CENTPGS------YHCTC-PTG---FVLLPDGKQC
LDLrAh:    C-----LDN----NGGCSHV----------CNDLKIG------YECLC-PDG---FQLVA-QRRC
LDLrArb:   C-----MRG----NGGCSHT----------CFDLRIG------HECHC-PKG---YRLV-DQRRC
EGFr3m:    CSS---PD-----NGGCSQI----------CLPLRPGS-----WECDCFP-G---YDLQSDRKSC
EGFr3h:    CSS---PD-----NGGCSQL----------CVPLSPVS-----WECDCFP-G---YDLQLDEKSC
EGFr4m:    CLYR------------NGGCEHI--------CQESLGTA----R--CLCR-EG---FVKAWDGKMC
EGFr4h:    CLYQ------------NGGCEHI--------CKKRLGTA-----W-CSCR-EG---FMKASDGKTC
LDLrCh:    C--ERTTL--S--NGGCNYL----------CLPAPQINPHSPKFTCAC-PDG---MLLARDMRSC
LDLrCrb:   C--EKTAL--P--NGGCQYL----------CLPAPQINSHSPKFTCAC-PDG---TLLAADMRSC
EGFr5m:    CG-------------PGGCGSHAR-------CVSDGETAE------CQCL-KG---F--ARDGNLC
```

# FIG. 22B

EP 0 293 936 B1

```
PC-4h:     CLVL--PLEHPCASLCCGHGT--------CIDGIGS------FSCDCR-SG---W----EGRFC
PC-4b:     CE-DR-PSGSPCDLPCCGRGK--------CIDGLGG------FRCDCA-EG---W----EGRFC
PS-4Bh:    CS-LK-PSI-------CGTAV--------CKNILGD------FECEC-PEGYR-YNLKS--KSC
PS-4Bb:    CV-LK-PSI-------CGTAV--------CKNIPGD------FECECA-EGYK-YNPVS--KSC
PS-5Bh:    CS-E----NM------C-AQL--------CVNYPGG------HTCYCD--GKKGFKLAQDQKSC
PS-5Bb:    CA-E----NL------C-AQL--------CVNYPGG------YSCYCD--GKKGFKLAQDQKSC
EGFr2m:    C-----PGNVSK----CSHG---------CVLTSDGP----R--CIC-PAG---SVLGRDGKTC
EGFr2h:    C-----PRNVSE----CSHD---------CVLTSEGPL------CFC-PEG---SVLERDGKTC
LDLrBh:    CQ-D--PDT-------CSQL---------CVNLEGG------YKCQCE-EG---FQLDPHTKAC
LDLrBrb:   CE-D--PDI-------CSQL---------CVNLAGS------YKCECR-AG---FQLDPHSQAC
EGFr6m:    CV-----LARSD----CPSTSSR------CINTEGG------YVCRCS-EG---YEG--DGISC
EGFr7m:    C--QR--GAHN-----CAENAA-------CTNTEGG------YNCTCA--GR---PSSP-GRSC
```

<div align="center">

# FIG.22C

</div>